# EUROPEAN PATENT SPECIFICATION

(11) **EP 1 537 231 B1**
(45) Date of publication and mention of the grant of the patent: **13.06.2007**
(21) Application number: 03795088.8
(22) Date of filing: 12.09.2003
(51) Int. Cl.: C12Q 1/00

(54) **AMP-ACTIVATED PROTEIN KINASE (AMPK) INHIBITOR SCREENING ASSAY**
ASSAY ZUM AUFFINDEN VON INHIBITOREN FÜR DIE AMP-AKTIVIERTE PROTEINKINASE (AMPK)
DOSAGE PAR CRIBLAGE POUR INHIBITEUR DE LA PROTEINE KINASE A ACTIVATION AMP (AMPK)

(30) Priority: 12.09.2002 GB 0221148
(43) Date of publication of application: 08.06.2005
(73) Proprietor: The University Court of the University of Dundee, Dundee DD1 4HN (GB)
(72) Inventor: HARDIE, Grahame, Wellcome Trust Biocentre, Dundee DD1 5EH (GB)
(74) Representative: Chapman, Paul Gilmour
(86) International application number: PCT/GB2003/003945
(87) International publication number: WO 2004/024942

(56) References cited:
- WO-A-01/20003
- WO-A-03/065795
- CHEUNG ET AL: "Characterization of AMP-activated protein kinase gamma-subunit isoforms and their role in AMP binding" BIOCHEMICAL JOURNAL, THE BIOCHEMICAL SOCIETY, LONDON, GB, vol. 346, no. 3, 15 March 2000 (2000-03-15), pages 659-669, XP002162237 ISSN: 0264-6021 cited in the application
- ARAD M ET AL: "CONSTITUTIVELY ACTIVE AMP KINASE MUTATIONS CAUSE GLYCOGEN STORAGE DISEASE MIMICKING HYPERTROPHIC CARDIOMYOPATHY" JOURNAL OF CLINICAL INVESTIGATION, NEW YORK, NY, US, vol. 109, no. 3, February 2002 (2002-02), pages 357-362, XP009023220 ISSN: 0021-9738 cited in the application
- GOLLOB M H ET AL: "PRKAG2 CARDIAC SYNDROME: FAMILIAL VENTRICULAR PREEXCITATION, CONDUCTION SYSTEM DISEASE, AND CARDIAC HYPERTROPHY" CURRENT OPINION IN CARDIOLOGY, GOWER ACADEMIC JOURNALS, LONDON, GB, vol. 17, no. 3, May 2002 (2002-05), pages 229-234, XP009023218 ISSN: 0268-4705
- DANIEL TYRONE ET AL: "Functional analysis of mutations in the gamma2 subunit of AMP-activated protein kinase associated with cardiac hypertrophy and Wolff-Parkinson-White syndrome." JOURNAL OF BIOLOGICAL CHEMISTRY, vol. 277, no. 52, 22 October 2002 (2002-10-22), pages 51017-51024, XP002268218 ISSN: 0021-9258
- DYCK J R B ET AL: "REGULATION OF 5'-AMP-ACTIVATED PROTEIN KINASE ACTIVITY BY THE NONCATALYTIC BETA AND GAMMA SUBUNITS" JOURNAL OF BIOLOGICAL CHEMISTRY, AMERICAN SOCIETY OF BIOLOGICAL CHEMISTS, BALTIMORE, MD, US, vol. 271, no. 30, 26 July 1996 (1996-07-26), pages 17798-17803, XP002907308 ISSN: 0021-9258
- SCHWAPPACH BLANCHE ET AL: "Golgi localization and functionally important domains in the NH2 and COOH terminus of the yeast CLC putative chloride channel Gef1p" JOURNAL OF BIOLOGICAL CHEMISTRY, vol. 273, no. 24, 12 June 1998 (1998-06-12), pages 15110-15118, XP002268319 ISSN: 0021-9258

## Description

The present invention relates to a method for screening for agents that bind to CBS domains such as found on AMP-activated protein kinase (AMPK), and agents identified using such an assay. Such agents which bind AMPK are candidates for use in the treatment of, for example, diabetes, obesity, hyperlipidaemia, and heart disease including cardiomyopathies caused by AMPK mutations.

The adenosine monophosphate (AMP)-activated protein kinase (AMPK) is the downstream component of a protein kinase cascade that is activated by rising AMP coupled with falling ATP, these changes in cellular nucleotides signalling a fall in cellular energy status. Following activation, the kinase switches on catabolic pathways while switching off many ATP-requiring processes, both through direct phosphorylation of metabolic enzymes and through effects on gene expression [1-3]. In 1999 it was proposed that activation of AMPK was a promising target for the development of new drugs aimed at treatment of Type 2 diabetes [4]. This view has been greatly strengthened by recent findings that the existing anti-diabetic drugs, metformin and rosiglitazone, activate AMPK *in vivo* and/or in cultured cells, albeit by distinct mechanisms [5-7]. Activators of AMPK are therefore potential new drugs aimed at treatment of Type 2 diabetes, with metformin and rosiglitazone being the prototypes. Since AMPK also inhibits fatty acid and triglyceride synthesis and stimulates fatty acid oxidation [1-3], and mediates at least some of the effects of the "satiety" hormone leptin [8], they may also be effective in treatment of obesity. Indeed, metformin treatment has been shown to reduce body weight and fat content, in obese, non-diabetic subjects [9].

AMPK exists as heterotrimeric complexes comprising a catalytic α subunit and accessory β and γ subunits. Each subunit occurs as multiple isoforms encoded by distinct genes (α1, α2, β1, β2, γ1, γ2, γ3) so that there are twelve possible heterotrimeric combinations [2]. AMP activates the complex via a complex multi-step mechanism that results in an ultrasensitive response, such that over the critical range of AMP concentrations there is a large activation in response to a small rise in AMP [10]. The effects of AMP are to: (i) bind to AMPK and cause allosteric activation; (ii) bind to AMPK and make it a better substrate for the upstream kinase, AMPKK; (iii) bind to AMPK and make it a worse substrate for protein phosphatases; (iv) allosterically activate the upstream kinase, AMPKK [11-13]. The simplest model to explain effects (i) through (iii) is that they are due to binding of AMP to a single allosteric site on the complex, although until now this has not been directly addressed experimentally. Effects (i) through (iii) are also antagonized by high concentrations of ATP [11-13], which may therefore compete for binding at the same site as AMP, although once again until now this has not been directly tested.

One way to screen for novel activators of AMPK is to purify the αβγ holoenzyme and conduct kinase assays in the presence and absence of candidate compounds. However this method has a number of disadvantages:
1) To date there is no published method for the expression of recombinant αβγ complexes. The only source of kinase complex for a high throughput screen (HTS) is therefore to purify the complex from a mammalian source such as rat liver. While possible, this is difficult, time-consuming and expensive to perform on a large scale.
2) Although the isoform composition for kinase complexes purified from a natural source such as rat liver can be determined, there is no large-scale method available to produce complexes that are homogeneous in terms of isoform content (e.g. α1β1γ1, α2β1γ2, etc.).
3) AMPK has a significant basal activity, with the natural agonist 5'-AMP typically producing only a 3- to 4-fold activation. The signal-to-noise ratio for this assay is therefore relatively poor.

Since AMPK activators are likely to be mimicking the effect of a natural activator such as 5'-AMP, an alternative method to develop novel compounds would be to identify the allosteric (AMP/ATP) binding domain(s), clone the DNA encoding them and express them as separate proteins, and carry out direct binding assays. Up to now the exact location of the AMP and ATP binding site(s) has not been clear, although there were indications that the γ subunits were involved [14]. Firstly, the degree of AMP dependence of the complex depended on the identity of the γ subunit isoform present, with the degree of activation by AMP increasing in the order γ3<γ1<γ2. Secondly, studies with the photoaffinity analogue, 8-azido-[³²P]AMP showed that it bound competitively with AMP at the allosteric site, and that on photo-activation it labelled the γ subunit. The three known isoforms of the γ subunit (γ 1, γ 2, γ 3) contain an N-terminal region of variable sequence and size, followed by four tandem repeats of a sequence motif known as a CBS domain. CBS domains contain about 60 amino acids and occur in several other proteins as well as AMPK [15]. CBS domains were originally identified by bioinformatic analysis as sequence motifs of ≈60 amino acids that occur in cystathionine β-synthase (CBS) and several other proteins, n all kingdoms of life from archaea to humans (15). Although their functions have been unknown, their importance was emphasized by findings that point mutations affecting certain key residues within them cause numerous hereditary diseases in humans. These include (with the protein/gene affected in parentheses): homocystinuria (cystathionine β-synthase (16)), retinitis pigmentosa (IMP dehydrogenase-1 (26, 27)), congenital myotonia, idiopathic generalized epilepsy, hypercalciuric nephrolithiasis and classic Bartter syndrome (chloride channels CLC1, CLC2, CLC5 and CLCKB respectively (28-31)) and the cardiac arrhythmia, Wolff-Parkinson-White syndrome (WPWS) (γ2 subunit of AMP-activated protein kinase (20-23)). They almost invariably occur in tandem pairs, although the γ subunits of AMPK are unique in having four. It has been suggested that they occur in pairs because two CBS domains are required to form a stable structure [15]. In no case are the functions of CBS domain known, although some clues have emerged from studies of the enzyme cystathionine β-synthase (from which the acronym "CBS" is derived). This enzyme metabolizes homocysteine and is activated by S-adenosyl methionine, and mutations in the gene cause the hereditary disease, homocystinuria, due to a toxic build up of homocysteine. Some human homocystinuria mutations map to the CBS domain, and these have been found to abolish activation by S-adenosyl methionine, suggesting that this domain represents the binding site for this adenosine derivative [16,17]. This is also supported by findings that proteolytic removal of the C-terminal region containing the CBS domain produces an enzyme that is active but is no longer regulated by S-adenosyl methionine [18]. Extrapolating these findings to AMPK, they raised the intriguing possibility that the CBS domains of the γ subunit isoforms might bind the adenosine moiety of AMP, consistent with our results obtained using 8-azido-AMP [14].

The CBS domains of the AMPK γ subunits have also become of increasing interest lately because mutations causing muscle abnormalities map to these domains. A mutation in the N-terminal CBS domain (CBS1) of γ 3 in Hampshire pigs causes abnormally high glycogen content in skeletal muscle [19], while six different point mutations in the coding region of the γ 2 subunit lead to cardiomyopathies in humans [20-23]. Intriguingly, the pig γ 3 mutation and three of the human γ 2 mutations (occurring in CBS1, CBS2 and CBS4), as well as one of the mutations in cystathione β-synthase causing homocystinuria [16] are in the equivalent positions when the sequences of the CBS domains are aligned. The effect of these mutations on the function of AMPK have until now been unclear.

The present inventors now demonstrate herein that tandem pairs of CBS domains from AMPK form the allosteric binding sites for AMP and ATP. DNAs encoding the CBS domains of the human γ 2 and γ1 isoforms have been cloned and expressed as GST fusions that either contain all four domains (CBS1-4), or the N-terminal pair (CBS1-2) or the C-terminal pair (CBS3-4). Furthermore, the binding of [¹⁴C]AMP or [³²P]ATP has been measured using a simple filter binding assay.

It is also demonstrated herein that the CBS1-4 construct competitively binds two molecules of AMP or ATP, displays positive co-operativity, and has affinities for these nucleotides consistent with their effects on the activity of the heterotrimeric complex. The CBS1-2 and CBS3-4 constructs both competitively bind single molecules of AMP or ATP. The mutations that give rise to cardiomyopathies all decrease the affinities for AMP and ATP, in some cases drastically. Moreover, the radioactive filter-binding assay can also be used to measure binding of non-radioactive compounds via competition with [¹⁴C]AMP. While this method is probably not ideal for a high-throughput screen (HTS) of compound libraries, it should be possible to adapt the assay into a format more suitable for an HTS.

WO01/20003 describes a screening method for identifying a substance capable of binding a mutated γ-subunit of AMPK.

Chaung et al (Biochemical Journal, The Biochemical Society, London, Volume 346, Number 3, 15 March 2000, pages 659-669) discloses the AMP dependence of different γ subunits of AMPK and their importance in the allosteric activation of the kinase when bound to AMP. The paper teaches the suggestion that binding cite for AMP might lie between the α and γ subunits.

The present invention is therefore based in part on the results of studies into the role of the γ subunit of AMPK in regulating AMPK function, in particular regulation by 5'-AMP and/or ATP.

It is amongst the objects of the present invention to provide a method of screening candidate agents which bind to the 5'-AMP and ATP binding site of AMPK, and which are expected to produce a modulatory effect on AMPK.

Thus, in a first aspect the present invention provides a method for identifying a substance capable of binding to the γ subunit of AMP-activated protein kinase (AMPK), or a fragment, derivative, homologue or mutant thereof, which method comprises:
a) providing a γ subunit polypeptide or a fragment, homologue, derivative or mutant thereof,
b) contacting the γ subunit polypeptide, a fragment, homologue, derivative or mutant thereof, with a test substance under conditions that would permit 5'-AMP and/or ATP to bind with the γ subunit polypeptide; and
c) determining whether said test substance has bound to the γ subunit polypeptide, a fragment, homologue, derivative or mutant thereof.

The substances identified by the above method may further be tested for their ability to modulate AMPK activity by administering the substance which has been determined to bind to the γ subunit polypeptide to AMPK, and determining any modulatory effect of the substance on AMPK. These substances may also be referred to as agonists or antagonists.

Said γ subunit, or a fragment, derivative, homologue or mutant thereof, may be in isolation from the other subunits associated with AMPK, or may be part of the multisubunit complex that forms the complete AMPK.

It is understood that any substance which binds to said γ subunit polypeptide of AMPK should modulate the activity of AMPK, or a fragment, derivative, homologue or mutant thereof, such as by affecting the binding of AMP and/or ATP. The term "modulation" refers to both positive and negative modulation. "Positive modulation", as used herein refers to an increase in the activity of AMPK, or a fragment, derivative, homologue or mutant thereof relative to the activity of AMPK in the absence or presence of 5'-AMP. "Negative modulation" as used herein refers to a decrease in the activity of AMPK, or a fragment, derivative, homologue or mutant thereof relative to the activity of AMPK in the absence or presence of 5'-AMP.

The term "AMPK" refers to the AMP-activated protein kinase EC 2.7.1.109. AMPK catalyses the phosphorylation of many metabolic enzymes under conditions that are well defined in the art [24] [e.g. buffer of neutral pH (7-7.4) in the presence of Mg²⁺ ions (e.g. 5 mM MgCl₂) and ATP (e.g. 0.2 mM), with or without AMP (e.g. 0.2 mM), which stimulates the reaction].

In general, the term "polypeptide" refers to a molecular chain of amino acids with a biological activity. It does not refer to a specific length of the products, and if required it can be modified *in vivo* and/or *in vitro,* for example by glycosylation, myristoylation, amidation, carboxylation or phosphorylation; thus inter alia peptides, oligopeptides and proteins are included. The polypeptides disclosed herein may be obtained, for example, by synthetic or recombinant techniques known in the art.

The term "γ subunit" refers one of the subunits of AMPK. There are three known γ subunits (γ 1, γ 2, γ 3) which each contain four tandem repeats of a sequence known as a CBS domain. The present inventors have demonstrated herein that the polypeptide fragment comprising the CBS domains 1-4 (CBS1-4) of the γ1, γ 2 and γ3 and subunits are capable of binding 5'-AMP and/or ATP. Thus, the γ subunit of the present invention may be any one of γ 1, γ 2 or γ 3, and, in particular, may for example be CBS domains 1-4 (residues 274-569 of γ1, 41-330 of γ1, 197-492 of γ3), or a fragment, homologue, derivative or mutant thereof. Furthermore, the present inventors have demonstrated that CBS domains 1 and 2, and separately, CBS domains 3 and 4 are also capable of binding 5'-AMP and ATP. Thus, the γ subunit of the present invention may also be CBS domains 1 and 2 (CBS1-2) (residues 41-177 of γ1, 274-410 of γ 2, 197-333 of γ3), or a fragment, homologue, derivative or mutant thereof, or CBS domains 3 and 4 (CBS3-4) (residues 197-330 of γ1, 430-556 of γ2, 353-492 of γ3). Moreover, the binding site of 5'-AMP and/or ATP in each of the domains are likely to be smaller than CBS1-2 or CBS3-4 in each of the γ subunits and in view of the information disclosed herein, may now be determined by routine experimentation. Thus, the term "fragments" refers to portions of a γ subunit which is capable of binding 5'-AMP and/or ATP. Examples include the domains CBS1-2 or CBS3-4 from the γ1, γ2 or γ3 subunits, or smaller polypeptide sequences of the domains, and all such fragments encompassed by the present invention.

The term "homologues" refers to variants of AMPK from different species which may not be identical in sequence but do have closely related functions. This is easily discerned by the skilled addressee by carrying out computer analysis of a postulated related sequence with a known AMPK in order to confirm whether or not it is a homologue. Enzymatic studies may also be conducted to confirm that a postulated homologue functions in a similar enzymatic manner to AMPK.

The term "mutants" refers to also known mutations in the γ subunit of the AMPK protein. For example, point mutations in the domain CBS1, which changes an arginine residue to a glutamine residue (R302Q), and, separately, in the domain CBS4, which changes an arginine residue to a glycine residue (R531G), have been implicated in hereditary heart disease. Therefore, by mutating the γ subunit in the assay of the present invention to mimic mutations associated with known disease states, substances may be isolated which are capable of modifying the activity of a mutant AMPK to wild-type levels.

It will be understood that for the particular polypeptides, homologues or mutants embraced herein, variations may occur due to polymorphisms that can exist between individuals or between members of the family. These variations may be demonstrated by (an) amino acid difference(s) in the overall sequence or by deletions, substitutions, insertions, inversions or additions of (an) amino acid(s) in said sequence. All such derivatives showing the recognised activity of wild-type AMPK are included within the scope of the invention. For example, for the purpose of the present invention conservative replacements may be made between amino acids within the following groups:
(I) Alanine, serine, threonine;
(II) Glutamic acid and aspartic acid;
(III) Arginine and leucine;
(IV) Asparagine and glutamine;
(V) Isoleucine, leucine and valine;
(VI) Phenylalanine, tyrosine and tryptophan.

Derivatives may also be in the form of a fusion protein wherein the γ subunit of AMPK, a homologue, derivative or mutant thereof is fused, using standard cloning techniques, to another polypeptide which may, for example, comprise a reporter protein, a transcriptional activation domain or a ligand suitable for affinity purification (for example glutathione-S-transferase or six consecutive histidine residues).

The γ subunit, fragments, derivatives, homologues or mutants thereof, of AMPK used in the assays may be obtained from mammalian extracts, produced recombinantly from, for example, bacteria, yeast or higher eukaryotic cells including mammalian cell lines and insect cell lines, or synthesised de novo using commercially available synthesisers. Preferably, the γ subunit, fragments, derivatives, homologues or mutants thereof, used in the assay is recombinant.

A substance which binds to the γ subunit, fragments, derivatives, homologues or mutants thereof, of AMPK and modulates the activity of AMPK may do so in several ways. It may act as a 5'-AMP analogue, thereby substantially activating AMPK. Alternatively, it may act as an ATP analogue, thereby substantially inhibiting AMPK. Candidate substances of this type may conveniently be screened by *in vitro* binding assays as, for example, described below. The modulation of AMPK activity can be tested *in vivo* using, for example the *in vivo* assays described below. The term *"in vivo"* is intended to encompass experiments with cells in culture as well as experiments with intact multicellular organisms.

The candidate substances may be used in an initial screen in batches of, for example 10 substances per reaction, and the substances of those batches that show binding activity tested individually. Candidate substances which show activity in *in vitro* screens such as those described below can then be tested in *in vivo* systems, such as mammalian cells which will be exposed to the substance and tested for altered function of AMPK using methods similar to those already defined in the art (e.g. [7]).

It is also possible to screen for compounds which selectively bind to the γ domain, especially the CBS domain over other domains. By selectively is meant greater than 10 fold, preferably 100 fold greater affinity. For example, a separate assay may be performed with for example, the kinase domain of AMPK in order to detect whether or not test agents which bind the γ or CBS domain also bind to the kinase domain. It may be that a compound which binds to the γ or CBS domain and activates AMPK may also bind to the kinase domain and potentially inhibit activity. By carrying out 2 such assays it may be possible to thereafter modify such a test agent so that the agent continues to bind to the γ or CBS domain and not the kinase domain. It will be appreciated that this would be extremely difficult or impossible using the whole enzyme.

As there are other enzymes which possess γ and/or CBS domains it may be preferable to assay a test agent on domains of these enzymes also. Thus, it may be desirable to ensure that any compounds identified as being able to modulate AMPK, do not influence the activity of other enzymes such as IMP dehydrogenase, CLC chloride channel and cystathionine β-synthase.

Thus, in a further aspect, the present invention provides a method of identifying a substance capable of binding to a CBS domain of a protein possessing such a CBS domain or domains, or a fragment, derivative, homologue or mutant thereof, which method comprises:
a) providing a CBS domain subunit polypeptide, or a fragment, homologue, derivative or mutant thereof;
b) contacting the CBS domain subunit polypeptide, or a fragment, homologue, derivative or mutant thereof, with a test substance under conditions that would permit 5'-AMP and/or ATP to bind with the CBS domain subunit polypeptide; and
c) determining whether said test substance has bound to the CBS domain subunit polypeptide.

The assays of the invention may be *in vitro* assays or *in vivo* assays, for example using an animal model. One type of *in vitro* assay for identifying substances which bind to the γ subunit, fragments, derivatives, homologues or mutants thereof, of AMPK may be a filter binding assay in which the substance and the protein are mixed in solution as described below. Other assays include those in which the substance or the protein may be in the solid phase (e.g. attached to a filter or membrane before addition of the binding partner), and methods involving scintillation proximity, changes in fluorescence of an intrinsic or attached fluorophore, surface plasmon resonance, isothermal titration calorimetry or nuclear magnetic resonance.

Candidate substances that are identifiable by the method of the invention as binding the γ subunit, fragments, derivatives, homologues or mutants thereof, of AMPK may be tested for their ability to modulate the activity of AMPK, or mutants thereof, using methods that have previously been described in the art (e.g. [24]).

In addition, an assay incorporating the identification of a candidate substance that binds to the γ subunit, fragments, derivatives, homologues or mutants thereof, of AMPK and the determination of any modulatory activity may preferably be used. Such assays include those involving reporter constructs in which the γ subunit, fragments, derivatives, homologues or mutants thereof are attached to a reporter where some property such as fluorescence, or the ability to regulate expression of a reporter gene, changes in response to binding of the substance.

Administration of candidate substances to cells may be performed by for example adding directly to the cell culture medium or injection into the cell. The assay is typically carried out *in vitro.* The candidate substance is contacted with the cells, typically cells in culture. The cells may be cells of a mammalian cell line.

The invention further provides a substance capable of modulating the activity of AMPK, or a fragment, derivative, homologue or mutant thereof, for use in treating the human or animal body by therapy or for use in diagnosis, whether or not practised on the human or animal body. Such a substance may thus be used in the prevention or treatment of, for example, diabetes, obesity, hyperlipidaemia and heart disease including cardiomyopathies caused by mutations in AMPK genes.

The formulation of a substance according to the invention will depend upon the nature of the substance identified but typically a substance may be formulated for clinical use with a pharmaceutically acceptable carrier or diluent. For example it may be formulated for topical, parenteral, intravenous, intramuscular, subcutaneous, intraocular or transdermal administration. A physician will be able to determine the required route of administration for any particular patient and condition.

Preferably, the substance is used in an injectable form. It may therefore be mixed with any vehicle which is pharmaceutically acceptable for an injectable formulation, preferably for a direct injection at the site to be treated. The pharmaceutically carrier or diluent may be, for example, sterile or isotonic solutions. It is also preferred to formulate that substance in an orally active form.

The dose of substance used may be adjusted according to various parameters, especially according to the substance used, the age, weight and condition of the patient to be treated, the mode of administration used and the required clinical regimen. A physician will be able to determine the required route of administration and dosage for any particular patient and condition.

The present invention will now be further described by way of example and with reference to the Figures which show:

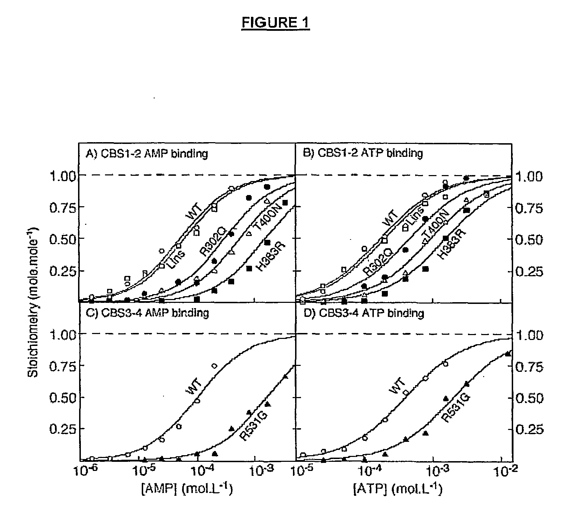

**Figure 1:** Shows binding of AMP (A and C) or ATP (B and D) by the CBS1-2 construct (A and B) or the CBS3-4 construct (C and D). The fusion proteins were either the wild type (WT, open circles), or one of five point mutants (R302Q, filled circles; Lins, open squares; H383R, filled squares; T400N, open triangles; R531G, filled triangles). Data were fitted to a single-site binding model: bound = [nucleotide] /(Kd + [nucleotide]), and the curves are theoretical curves obtained using the values for Kd shown in Table 1;

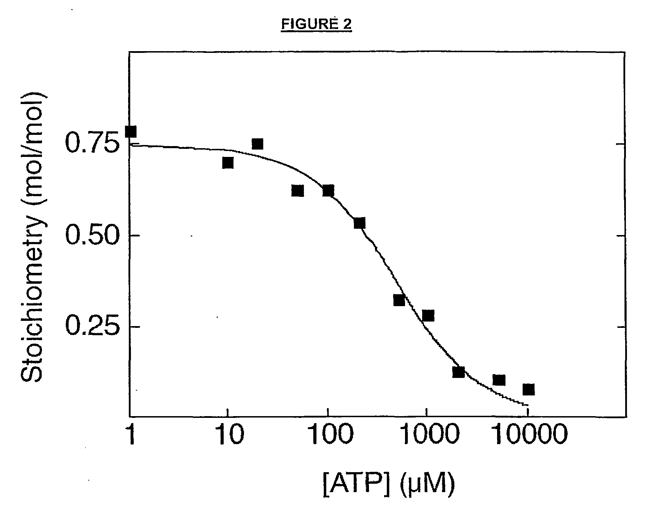

**Figure 2:** Shows displacement of AMP from the CBS1-2 construct by ATP. A fixed concentration of [¹⁴C]AMP (180 µM) was incubated with the CBS1-2 construct in the presence of increasing concentrations of ATP. Data were fitted to the binding model: Bound =AMP/(AMP+Kd_{AMP}(1+[ATP]/Kd_{ATP}))

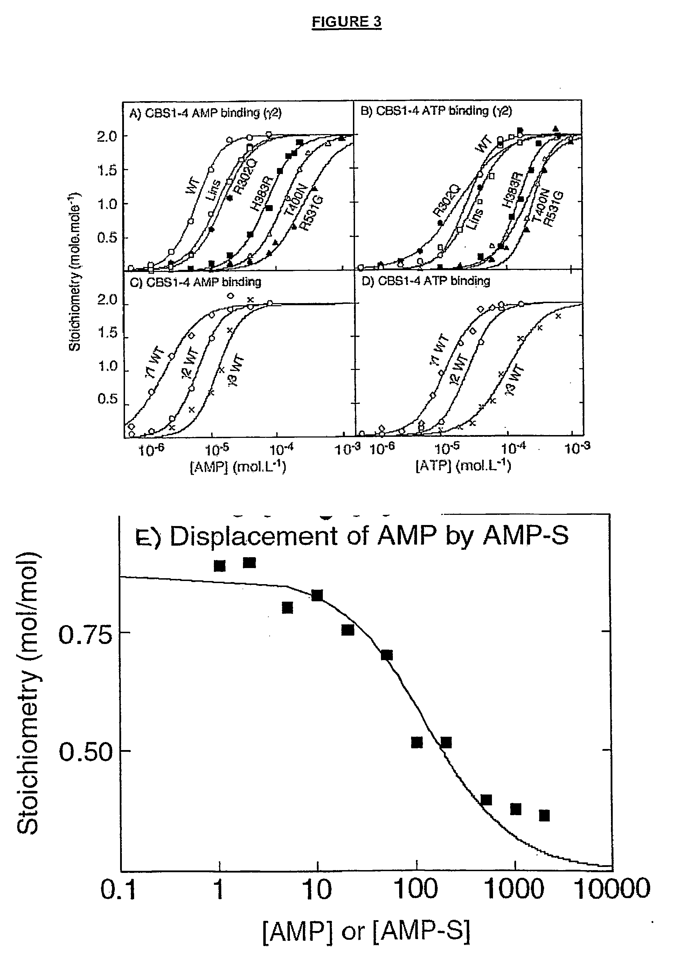

**Figure 3:** shows (A) Binding of AMP; and (B) ATP to the GST-CBS1-4 fusion protein from γ2 and WPWS mutants; (C) binding of AMP; and (D) ATP by the GST-CBS1-4 fusion proteins from γ1, γ2 and γ3. The methodology was as for Fig. 1, except that data were fitted to a two site Hill plot model: bound = 2 x [nucleotide]^{h}/(B_{0.5}^{h} + [nucleotide]^{h}); (E) shows displacement of AMP by AMP-S;

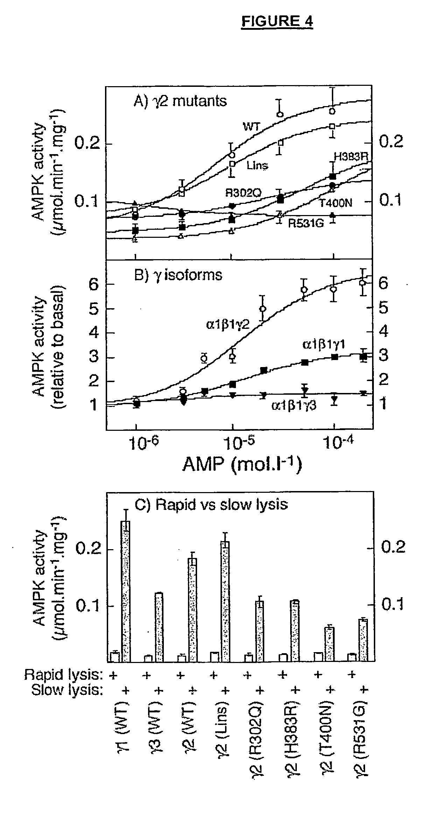

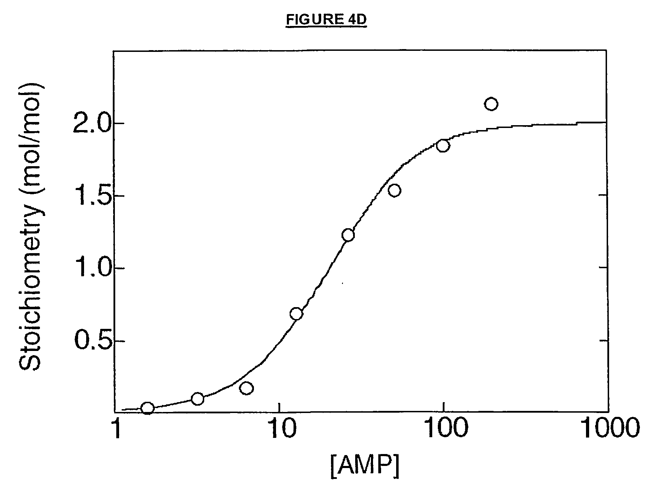

**Figure 4:** shows (A) Activaton of recombinant α1β1γ2 heterotrimers, with or without WPWS mutations, by AMP; (B) activation of recombinant α1β1γ1, α1β1γ2 and α1β1γ3 heterotrimers by AMP; (C) activation of α1β1γ2 heterotrimers, with or without WPWS mutations, by slow versus rapid lysis. Plasmids expressing *myc*-tagged α1, β1 and γ1, γ2 (with or without WPWS mutations) or γ3 were expressed in CCL13 cells and the recombinant complexes immunoprecipitated using anti-myc antibodies. AMPK activity was then determined at various concentrations of AMP. In A) and B), the cells were harvested by slow lysis to elicit maximal phosphorylation (32); in C) the cells were harvested by rapid or slow lysis and the assays conducted at 200 µM AMP. Results are means ± standard error for duplicate immunoprecipitations.; (D) illustrates the binding of AMP and to the purified CBS1-4 domains from γ1. The curves were obtained by fitting the data to the equation *Bound* = *2 x [AMP]^{h}*/*(B_{0.5}^{h}+[AMP]^{h})* and yielded the estimates for *Kd* and *h* quoted in the text;

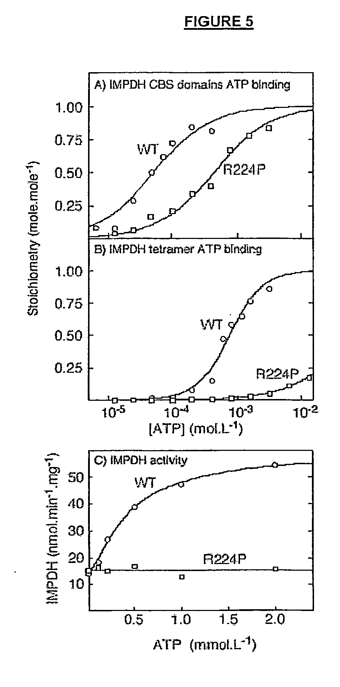

**Figure 5:** shows binding of ATP by GST fusions of (A) the isolated CBS domain pair (residues 112-232); and (B) full-length IMPDH2 (residues 1-514); and (C) activity of full-length IMPDH2 as a function of ATP concentration. Results were obtained both for the wild type sequence and an R224P mutation. Data in (A) and (B) were fitted to a single site binding model as for Fig. 1. Data in (C) were fitted to the model: activity = Basal+(((StimulationxBasal)-Basal)x[ATP]^{h})/(Ka^{h}+[ATP]^{h});

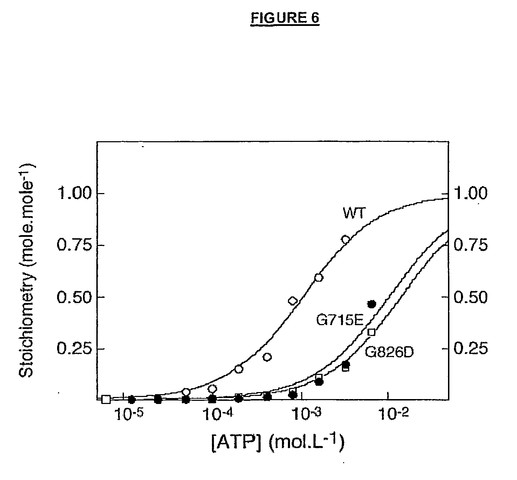

**Figure 6:** shows binding of ATP by a GST fusion of the isolated CBS domain pair (residues 582-840) from CLC2, and of G715E and G826D mutations. Data were fitted to a single site binding model as for Fig. 1;

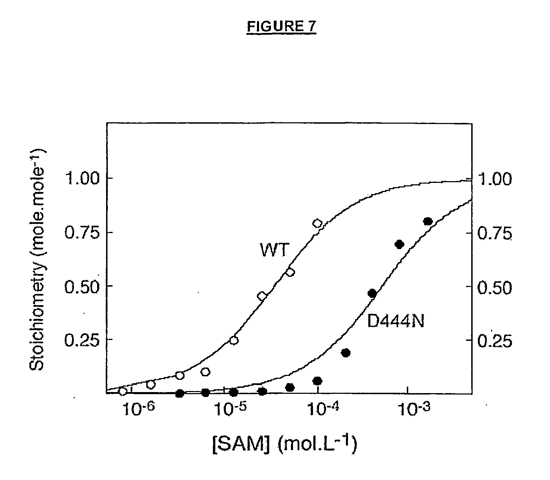

**Figure 7:** shows binding of S-adenosyl methionine by a GST fusion of the isolated CBS domain pair (residues 416-551) from cystathionine β-synthase, and of a D444N mutation. Data were fitted to a single site binding model as for Fig. 1; and

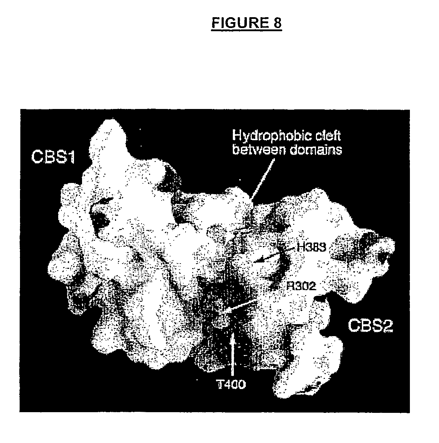

**Figure 8:** shows a model of the N-terminal pair of CBS domains (CBS1 and CBS2) from the γ2 subunit of AMPK. The picture is a view of a molecular surface representation made using the program GRASP (50), with CBS1 on the left. The model was made using MODELLER6 (51), and was based on the atomic co-ordinates of the CBS domain pair from a bacterial IMP dehydrogenase (33) (PDB code 1ZFJ). Electrostatic potential at the surface is depicted as red for negative and blue for positive. The approximate position of residues mutated in WPWS are shown. R302 and H383 occupy equivalent positions in CBS1 and CBS2 respectively, and are adjacent to each other in the model. Part of residue T400 projects into the hydrophobic cleft between the two domains.

### EXAMPLES

### Materials

Glutathione-Sepharose, [¹⁴C]-AMP, and [γ-³²P] ATP were from Amersham-Pharmacia Biotech. The Pfu Turbo PCR kit, QuikChange Site-directed Mutagenesis kit, and XL-1 Blue E. *coli* were from Stratagene. BL21 (DE3), TOP 10F' *Escherichia coli* and 4-12% Bis-Tris Polyacrylamide gels were obtained from Invitrogen. Restriction enzymes were from Promega. Synthetic oligonucleotides were from Sigma-Genosys. MF Filter Membrane Discs were from Millipore and P81 phosphocellulose from Whatman. AMP and ATP were from Roche Diagnostics. All other chemicals were from Sigma.

### Construct Preparation

DNA fragments were produced encoding all four (CBS1-4), or the first two (CBS1-2), or the last two (CBS3-4) of the tandem CBS domains from the human AMPK γ2 subunit. DNAs were produced by the polymerase chain reaction (PCR) using the Stratagene Pfu Turbo PCR kit and a human γ2-encoding pcDNA3 plasmid [14] as template. The following oligonucleotides were used as primers: (CBS1-4), 5'-CGC GGA TCC GCG GTT CCA ACC AGT TCA AAG CTT GTT-3' (sense), 5'-CCG CTC GAG CGG CTC CGT TTC TGT CTC CTT TTG TTT-3' (antisense); CBS1-2 (sense strand as CBS1-4), 5'-CCG CTC GAG CGG AAG CTG GAG GAA CTT GAG GAT TCT-3' (antisense); CBS3-4: 5'-CGC GGA TCC GCG ATA GGA ACG TAC CAC AAC ATT GCC-3' (sense), 5'-CCG CTC GAG CGG GAG GAT CAG GGC TTG CAG AAT GTC - 3' (antisense). Human γ3 (residues 197-479) was also used as a template for PCR (14). The PCR products were subcloned into pGEX-KG as *5'-BamHI* and *3'-XhoI* restriction fragments for expression as glutathione-S-Transferase (GST) fusions in E. *coli.*

DNA encoding the CBS1-4 domains of the γ1 isoform was produced using PCR with a rat γ1-encoding pcDNA3 plasmid [25] as template. The primers used were: 5'-CGC GGA TCC GCG ATG GAG ACG GTC ATT TCT TCA-3' (sense), 5'-CCG CTC GAG CGG GGG CTT CTT CTC TCC ACC TGT-3' (antisense). The PCR products was sub-cloned into pGEX-KG as above.

For IMPDH2, DNAs encoding residues 112-232 or the full-length protein (1-514) were amplified using human liver cDNA (Clontech) as template, and cloned into pHAT20 (Clontech) using 5'-*AgeI* and *3'-KpnI* sites added to the primers. For the other constructs, DNAs encoding residues 582-840 of CLC2, or 416-551 of cystathionine β-synthase, were amplified using human liver cDNA as template, and cloned into pGEX-KG as above using *5'-BamHI* and *3'-XhoI* sites added to the primers. All mutations were created using the Stratagene QuikChange Site-directed Mutagenesis system.

### Purification of recombinant CBS domains

An overnight culture of BL21 (DE3) E. *coli* containing the appropriate construct was used to inoculate 1 L of LB-ampicillin (50µg/µl) at a ratio of 1:100 (v/v). The culture was grown until the A₆₀₀ reached 0.4, after which IPTG was added to a final concentration of 1 mM. The cells were incubated for a further 3 hr with shaking at 37°C. Pellets were resuspended in 20 ml of PBS pH 7.4 containing 1 mM EDTA, 5 mM dithiothreitol, 1 mg/ml lysosyme and Complete Protease Inhibitor Cocktail (Roche). Following incubation on ice for 30 min, the cells were lysed in 1.5% sarkosyl, with cellular debris subsequently cleared by ultracentrifugation at 4°C for 1 hr. Triton X-100 was added to the cleared supernatant to a final concentration of 2% (w/v). The lysate was applied to a 5 ml glutathione-Sepharose column pre-equilibrated with 5 column vols of PBS. The beads were washed with 5 column vols of PBS containing 1 M NaCl followed by 5 column vols of PBS. Protein was eluted with 20 ml of PBS pH 7.4 containing 20 mM reduced glutathione. Protein was detected by the Bradford assay and SDS-PAGE analysis. Pooled fractions were dialysed against TBS pH 7.4 and concentrated using centrifugal ultrafiltration (Bismax-10K, Milipore).

The IMPDH2 constructs were His-tagged and were purified on chelating-Sepharose (Pharmacia) charged with 50 mM CoCl₂ according to manufacturer's instructions.

### Expression of AMPK heterotrimers in CCL13 cells and kinase assays.

PCDNA plasmids (14,25,32) encoding α1, β1 and γ1, γ2 or γ3 were expressed in CCL13 cells (25), subject to "rapid lysis", immunoprecipitated using anti-myc antibody, and assayed as described previously (33). "Slow lysis" of CCL13 cells was as described by Stein et al (34).

### Ligand-binding assays.

Various concentrations of [¹⁴C]-AMP, [γ-³²P] ATP or [³⁵S]SAM were incubated with GST-CBS domain fusions (1 µM for WT, up to 20 µM for some mutants) for 10 min at 25°C in a 20 µl total volume in TBS. Reactions were stopped by spotting 10 µl onto a Millipore MF Filter Membrane disc and rapidly washing under high vacuum with 1 ml of ice-cold TBS. Radioactivity was determined on the filters by scintillation counting. Non-specific binding (which was trivial) was determined by performing the assay with GST as a control, and was subtracted. For measurements of ATP binding, 5 mM MgCl₂ was routinely included; it was omitted for studies of AMP binding, although separate assays showed that MgCl₂ did not affect the binding of either nucleotide. Data were fitted to the binding models given in the text, using GraphPad Prism 3. Estimates of parameters resulting from fits are quoted in the text and Table 1 ± standard error, and the curves shown in figures are theoretical curves obtained using those estimates.

### Enzymatic assay of IMPDH2.

IMPDH2 was assayed by following the increase in absorbance at 340 nm due to formation of NADH in a BMG Fluostar Optima plate reader at 30°C in plate mode. The assay buffer contained 50 mM Tris/HCl, 100 mM KCl, 1 mM DTT, 3 mM EDTA, and 5 mM MgCl₂. Where ATP was added, additional MgCl₂ was added to maintain a constant 5 mM excess of MgCl₂ over ATP. The total volume was 200µl and the reaction started by addition of IMP (or NAD⁺ where [IMP] was the variable).

### Results

### Expression of CBS domains as GST fusions in Escherichia coli

The boundaries of the CBS domains were taken from the alignments by Bateman [15]. Using a plasmid encoding human γ2 as the template [14], we used PCR to produce DNAs encoding all four CBS domains (CBS1-4, residues 274-569), the N-terminal pair (CBS1-2, residues 274-410) or the C-terminal pair (CBS3-4, residues 430-556). The PCR primers had been designed to create *BamHI* and *XhoI* sites at the ends, and these were used to insert them into a vector that created a fusion protein with glutathione-S-transferase at the N-terminal end followed by a hexaglycine spacer, with the CBS domains at the C-terminus. All three constructs were expressed in E. *coli* and could be purified on glutathione-Sepharose as soluble proteins of the expected sizes in good yield (CBS1-4, 61 kDa, ≈5 mg/L of culture; CBS1-2, 43 kDa, ≈2 mg/L; CBS3-4, 43 kDa, ≈2 mg/L).

### Binding of adenine nucleotides by the CBS1-2 construct

The binding of AMP to the CBS1-2 domain construct was assessed using [¹⁴C]AMP and a filter binding assay. Initial fitting of the data to the binding model [Bound = Bₘₐₓ × [AMP]/(Kd +[AMP])] yielded a maximal binding (Bₘₐₓ) of 0.95 ± 0.05 (± standard error) moles of AMP per mole of protein. As this is not significantly different from one, the data were subsequently fitted to a simple single-site binding model [Bound = [AMP]/(Kd +[AMP])], yielding a dissociation constant (Kd) of 53 ± 5 µM (Fig. 1A). The Kd for AMP was not affected by the presence or absence of 5 mM MgCl₂ (not shown). We also used site-directed mutagenesis to create the point mutation in CBS1 that causes hereditary heart disease (R302Q). The mutant still bound 1 molecule of AMP but the Kd increased 5.7-fold to 303 ± 42 µM (Fig. 1A).

The present inventors also used site-directed mutagenesis to create four of the mutations in CBS1-2 that cause WPWS, i.e. R302Q, H383R and T400N, and Lins (which inserts a leucine residue in the linker between CBS1 and CBS2). All mutants still bound 1 molecule of AMP but the Kd values for R302Q, T400N and H383R were increased 6-, 10- and 28-fold relative to wuld-type, respectively (Fig. 1A, Table 1). The Kd for Lins was not significantly different from the wild type.

Since high concentrations of MgATP antagonize activation of AMPK by AMP [11], we suspected that the CBS1-2 construct would also bind MgATP. Experiments with [γ-³²P]ATP in the presence of MgCl₂ using the filter binding assay revealed that this was indeed the case, and the data could be fitted to the simple single-site binding model with a Kd of 175 ± 17 µM, 3.3-fold higher than the Kd for AMP. The 302Q mutant still bound 1 molecule of ATP but the Kd increased 2.6-fold to 468 ± 86 µM (Fig. 1B). All of the mutants still bound ATP but the Kd values were increased in the same rank order, albeit to a lesser extent, than for AMP binding. For R302Q, T400N and H383R the increases were 3-, 5- and 9-fold respectively, while Lins was again not significantly different from the wild type (Fig. 1B. Table 1).

To determine whether binding of AMP and ATP was competitive, we measured the effect of increasing concentrations of unlabelled MgATP on the binding of a fixed concentration of [¹⁴C]AMP that gave 75% of maximal binding. High concentrations of MgATP completely displaced binding of [¹⁴C]AMP (Fig. 2A), suggesting that the binding was competitive or mutually exclusive. The data was fitted to a simple single site competitive binding model [Bound = [AMP]/((Kd_{AMP}(1 + [ATP]/Kd_{ATP})) +[AMP])]. This yielded estimates for Kd_{AMP} and Kd_{ATP} of 51 and 180 µM. These are very close to the estimates obtained by direct binding measurements (53 and 175 µM).

### Binding of AMP and AMP-S by the CBS3-CBS4 construct

The binding of AMP to the CBS3-4 domain construct was assessed using [¹⁴C]AMP and a filter binding assay. The results were consistent with the presence of a single AMP binding site, and fitting of the data to the equation [Bound = [AMP]/(Kd +[AMP])] yielded a Kd of 104 ± 10 µM. We also used site-directed mutagenesis to create a point mutation in CBS4 that causes hereditary heart disease (WPWS) (R531G). This mutant still bound 1 molecule of AMP but the Kd increased 15-fold to 1570 ± 110 µM (Fig. 1C, Table 1). The wild type also bound ATP with a Kd 4-fold higher than that for AMP, while the Kd for ATP was increased 5-fold relative to the wild-type in the R531G mutant (Fig. 1D, Table 1).

### The four tandem CBS domains from AMPK- γ2 bind two molecules of AMP or ATP with positive co-operativity.

Since the CBS1-2 and the CBS3-4 fusion proteins from γ2 each bound one molecule of AMP or ATP, we expected that the CBS1-4 construct would bind two, which was indeed the case. The best fits were obtained using a two identical, interacting site (Hill plot) model, i.e. *bound* = 2 × *[AMP]^{h}*/*B_{0.5} ^{h}+[AMP]^{h})* (Fig. 3A, Table 1). For the wild type γ2 construct, this yielded a B_{0.5} (concentration giving half-maximal binding) for AMP of 61 µM and a Hill coefficient (h) very close to 2. The Hill coefficient indicates that the two sites bind AMP with strong positive co-operativity, i.e. that once AMP is bound at the first site the affinity at the second site is increased so that it fills almost immediately. Similar results were obtained for the binding of ATP (Fig. 3B, Table 1), which yielded a B_{0.5} >4-fold higher than that for AMP, consistent with the finding that both CBS1-2 and CBS3-4 bound ATP with lower affinity than AMP. All WPWS mutants still bound 2 molecules of AMP with positive co-operativity, but the B_{0.5} values were markedly increased (Fig. 3A, Table 1), even with the *Lins* mutant where we did not find a significant difference with the CBS1-2 construct. For Lins, R302Q, H383R, T400N and R531G the increases in B_{0.5} were 2.1-fold, 2.6-fold, 12-fold, 22-fold and 46-fold respectively. All mutants also bound 2 molecules of ATP, but with changes in B_{0.5} and/or Hill coefficient (Fig. 3B, Table 1). With the H383R, T400N and R531G mutants there were large increases in B_{0.5} (6-, 8-and 10-fold) with no significant change in Hill coefficient. For the R302Q mutant there was actually a slight decrease in B_{0.5}, but this was accompanied by a decrease in Hill coefficient (from 2.2 to 1.3): There was no significant difference in ATP binding between the wild type and the Lins mutant.

To show that the competitive binding assay described above for the CBS1-2 construct could be used as a screening assay to measure binding of non-radioactive AMP analogues other than ATP, we studied binding of adenosine 5'-O-monothiophosphate (AMP-S) to CBS3-4. This synthetic analogue, in which one of the oxygen atoms on the phosphate group is replaced by sulphur, is an activator of AMPK with a potency even greater than that of AMP itself (concentration giving half-maximal activation = 3.1 µM vs 4.7 µM for AMP, data not shown). Increasing concentrations of AMP-S displaced binding of AMP from the CBS3-4 construct, and fitting to the single site competitive binding model [Bound = [AMP]/((Kd_{AMP}(1 + [ATP]/Kd_{AMP-S})) +[AMP])] yielded a Kd_{AMP-S} of 48 ± 12 µM (Fig. 3E), which is 2-fold lower than the Kd of CBS3-4 for AMP.

### Effects of WPWS mutations on activation of AMPK heterotrimers by AMP.

While the results in the previous section showed that isolated CBS domain pairs can bind AMP and ATP, they did not conclusively prove that these domains form the regulatory nucleotide binding sites in the heterotrimeric AMPK complex. To address this, we expressed recombinant α1β1γ2 heterotrimers in wild type and mutated forms in CCL13 cells, purified them by immunoprecipitation via *myc* epitope tags on the α subunit, and assayed at various AMP concentrations. Fig. 4A shows that for the Lins, R302Q, H383R, T400N and R531 G mutations the A_{0.5} values (concentration causing half-maximal activation) for AMP increased in the same order in which these mutations increased the B_{0.5} for binding of AMP to the isolated CBS1-4 constructs, i.e. wild type (7±3 µM) ≈ *Lins* (9±4 µM) <R302Q (23±6 µM) <H383R (51±40 µM) <T400N (95±32 µM) <R531G (>100 µM). The A_{0.5} values for the heterotrimers were all around 10-fold lower than the B_{0.5} values for the isolated CBS1-4 constructs, indicating that the presence of the α, and/or, β subunits increases the affinity for AMP. It was not possible to conduct assays above 100 µM AMP because at these concentrations the nucleotide inhibits the activity by competing with ATP at the kinase domain on the α subunit (data not shown). For this reason, the estimates of A_{0.5} for the H383R and T400N mutants are somewhat less accurate than the others, while no value could be obtained for the R531 G mutant. The latter appeared to have a slightly elevated basal activity in the absence of AMP, and was actually *inhibited* by adding AMP. With some mutants, the maximal activation also appeared to be affected. For example, the R302Q mutant was only stimulated 3-fold by AMP whereas the wild type was stimulated 6-fold.

### Effects of identity of γ isoform on activation of AMPK heterotrimers by AMP.

Fig. 4B shows that when the activation of the wild type α1β1γ1, α1β1γ2 and α1β1γ3 heterotrimers were compared using the same methodology, they differed in the degree of stimulation by AMP rather than in the A_{0.5} for AMP. The α1β1γ1, α1β1γ2 and α1β1γ3 heterotrimers were stimulated 3.3-, 7.0- and 1.5-fold by AMP, but the A_{0.5} values were similar (13±2,12±3 and 2±2 µM respectively). The value of A_{0.5} for α1β1γ3 is approximate only, due to the very small degree of stimulation by AMP with that isoform.

### γ2 mutations do not cause constitutive activation of AMPK.

It has previously been claimed that mutations that are associated with WPWS cause constitutive activation of AMPK (23, 35). To address that, we examined the activation of expressed AMPK in CCL13 cells by "slow lysis" as opposed to "rapid lysis". "Slow lysis" involves harvesting the cells by scraping them off and centrifuging them prior to resuspension in homogenisation medium, and activates AMPK by a combination of mechanical stress, hypoxia and/or glucose deprivation. "Rapid lysis" involves pouring off the medium and lysing the cells *in situ* on the culture dish using ice-cold lysis buffer, and we believed this preserves much better the phosphorylation status of AMPK. Fig. 4C shows that the stress of slow lysis significantly activated the α1β1γ1, α1β1γ2 and α1β1γ3 hetrotrimers, although the degree of activation was lower with the α1β1γ3 complex. All of the γ2 mutants were also activated, although the degree of activation of the R302Q, H383R, T400N and R531 G mutants was significantly lower than that of the wild type and the *Lins* mutant. Most significantly, there was no evidence for constitutive activation of any of the mutants when the cells were harvested by rapid lysis.

### Binding of adenine nucleotides to a CBS1-4 construct derived from the γ1 isoform

To demonstrate that our method is also applicable to other isoforms of the γ subunit, we constructed a GST fusion of the four CBS domains derived from the γ1 rather than the γ2 isoform. The construct was expressed in E. *coli* and could be purified on glutathione-Sepharose as a soluble protein of the expected size (62 kDa) in good yield (≈3 mg/L of culture). As expected, this protein bound two molecules of AMP with positive co-operativity, and fitting to the two site Hill plot equation [Bound = 2 x [AMP]^{h}/(B_{0.5}^{h}+[AMP]^{h})] yielded a B_{0.5} of 20 ± 1 µM and a Hill coefficient of 1.6 ± 0.1 (Fig. 4D). Thus, the γ1 isoform has a 3-fold higher affinity (3-fold lower B_{0.5}) for AMP than γ2.

### The CBS domains from IMP dehydrogenase bind ATP, and this is impaired by a mutation causing retinitis pigmentosa.

To examine whether binding of adenine nucleotides is a general function for CBS domains, or is specific to the, γ subunits of AMPK, we also studied IMP dehydrogenase (IMPDH). Retinitis pigmentosa can be caused by an R224P mutation in the second CBS domain of the IMPDH1 isoform (27). However, the IMPDH2 isoform is 84% identical in amino acid sequence with IMPDH1, and the arginine mutated in retinitis pigmentosa is conserved. Because the IMPDH2 isoform is ubiquitously expressed and a suitable cDNA to use as a template for PCR was more readily available, we cloned DNA encoding the single pair of CBS domains from human IMPDH2, created an R224P mutation, and expressed both the wild type and the mutant as His-tagged proteins in E. *coli.* The wild type fusion protein bound 1 molecule of ATP with a Kd of 54 µM, while with the R224P mutant the Kd increased >8-fold (Fig. 5A, Table 1). Using displacement of bound ATP as the assay, the wild type protein also bound AMP or GMP, but only at concentrations that are supra-physiological (Kd = 440 and 5760 µM respectively; data not shown).

We also cloned and expressed full length IMPDH2 and examined the binding of ATP to it (Fig. 5B). Interestingly, this protein (which is a tetramer) bound ATP with a B_{0.5} of 0.76 mM, 14-fold higher than the Kd for the isolated CBS domains, and much closer to the physiological range of ATP concentrations. The binding of ATP to full length IMPDH2, but not to the isolated CBS domains, also displayed positive co-operativity, with a Hill coefficient of 1.7. ATP binding by the full-length tetramer was also drastically affected by the R224P mutation, with a >80-fold increase in B_{0.5} (Fig. 5B, Table 1).

### ATP is an allosteric activator of IMP dehydrogenase.

Since we could not find any published reports describing effects of ATP on IMPDH activity, we assayed the recombinant enzyme in the presence and absence of the nucleotide. Fig. 5C shows that ATP stimulated IMPDH activity >4-fold with a half-maximal effect at 0.44 ± 0.05 mM and a Hill coefficient of 1.3 ± 0.1. This is close to the B_{0.5} and Hill coefficient obtained for ATP binding to the tetramer. The effect of ATP appeared to be an effect on Vmax, rather than on the Km values for the substrates IMP and NAD⁺ (not shown). Even at the highest concentration used (2 mM), ATP had no effect on the R224P mutant version of the fulllength tetramer (Fig. 5C). This is consistent with the insignificant level of binding of ATP to the tetramer at this concentration (Fig. 5B).

### The CBS domain pair from the chloride channel CLC2 bind ATP and this is severely affected by pathogenic mutations.

We also cloned and expressed DNA encoding the CBS domain pair from the chloride channel CLC2. Fig. 6 shows that this construct bound one molecule of ATP with a Kd of 1.06±0.08 mM, while G715E and G826D mutations were associated with 10- and 14-fold increases in the Kd. The G715E mutation, in the linker between the first and second CBS domains of CLC2, is associated with idiopathic generalized epilepsy (29), while the G826D mutation is in an equivalent position in the second CBS domain to the G859D mutation in CLC1 that is associated with congenital myotonia (28).

### The CBS domain pair from cystathionine β-synthase bind S-adenosyl methionine and this is affected by a homocystinuria mutation.

Finally, we cloned and expressed DNA encoding the C-terminal CBS domain pair from the enzyme cystathionine β-synthase. This domain pair bound one molecule of S-adenosylmethionine (SAM) with a Kd of 34±2 µM, while the D444N mutation that causes homocystinuria (16) increased the Kd for SAM by 15-fold to 510±70 µM (Fig. 7).

### Discussion

Our results strongly suggest that the four tandem pairs of CBS domains in the γ subunits of AMPK provide the allosteric binding sites for AMP and ATP in the whole αβγ AMPK complex. The key evidence in favour of this may be summarised as follows:
1) Previous evidence using the photoaffinity analogue 8-azido-[³²P]AMP (where the photo-activatable azido group is attached to carbon 8 of the adenine) suggested that the adenine moiety of AMP bound to the γ subunit [14].
2) Although AMPK complexes containing γ1, γ2 or γ3 are all activated by AMP (albeit to different extents [14]), the only region where the sequences of these three isoforms are conserved are the four CBS domains.
3) We show herein that pairs of CBS domains expressed as GST fusions bind AMP and ATP with binding constants of the same order of magnitude as the concentrations of these nucleotides that cause activation (AMP) or inhibition (ATP) of AMPK. We originally reported that AMPK was activated by AMP with a half-maximal effect (A_{0.5}) at 4.4 µM when assays were carried out in 200 µM ATP, increasing to 29 µM at 4 mM ATP [11]. This was performed with rat liver AMPK, which contains primarily the γ1 isoform. The B_{0.5} (concentration of AMP giving half-maximal binding) to the CBS1-4 construct from γ1 was 20 ± 1 µM, 3-fold lower that that for γ2 and of the same order of magnitude as the A_{0.5} for the rat liver complex. Because ATP binds at both the catalytic and the allosteric sites, the inhibitory effects of ATP are more difficult to study in a quantitative manner with the whole αβγ complex, but it was clear from previous data [11] that the affinity for ATP at the allosteric site was less than that for AMP, consistent with our findings on AMP and ATP binding.
4) Point mutations in the CBS domains that give rise to cardiomyopathies, while not abolishing binding of AMP and ATP, cause large decreases in the affinity for these nucleotides. While the effects of these mutations on the regulation of the αβγ holoenzymes have not yet been reported, such large decreases in affinity at the regulatory sites for nucleotides would be expected to severely affect the function of AMPK in the heart under conditions of metabolic stress. Our results are therefore consistent with findings that the mutations greatly increase the risk of sudden death from heart failure.

The most unexpected finding of this study was that the four CBS domains of the γ2 subunit bind two molecules of AMP or ATP. Although we now show that the four CBS domains of γ2 bind AMP and ATP with positive co-operativity, no evidence for positive co-operativity was noticed in previous studies of activation of the whole complex by AMP or ZMP [e.g. [11]]. However, there are several technical reasons why this might previously have been missed. Firstly, AMPK complexes exhibit a basal activity even in the absence of added AMP, so that the background is much higher than in our binding assays. Secondly, kinase assays require the presence of MgATP that competes with binding of AMP at the allosteric site. Thirdly and most importantly, AMP inhibits activity at high concentrations (probably because it competes with binding of ATP at the catalytic domain); this would depress the activity at high AMP concentrations and thus reduce the apparent degree of positive co-operativity. Binding of nucleotides to CBS1-4 is not complicated by any of these factors, making positive co-operativity much easier to demonstrate.

Why AMPK should have two allosteric sites for AMP and ATP remains unclear, although because of the positive co-operativity of binding this would further increase the sensitivity of the system, such that a small increase in concentration of the activating or inhibiting nucleotide would produce a large response. Our current hypothesis is that binding of AMP at the first site greatly increases the affinity at the second site, but that both sites must be occupied by AMP to generate active kinase.

These findings provide the first indications that the different isoforms of AMPK have different affinities for AMP (B_{0.5} for CBS1-4 from γ1 and γ2 were 20 ± 1 and 61 ± 2 µM respectively). In a tissue like skeletal muscle, which expresses both γ1 and γ2, this would allow the system to respond to a wider range of AMP concentrations than would otherwise be the case.

Overall, our results show that tandem pairs of CBS domains form allosteric binding sites for adenosine derivatives, i.e. AMP (and, at higher concentrations, ATP) in the case of AMPK, ATP in the cases of IMP dehydrogenase and CLC chloride channels, and Sadenosyl methionine in the case of cystathionine β-synthase. Moreover, they show that the many mutations in CBS domains that cause human hereditary diseases invariably impair the binding of these regulatory adenosine derivatives. Our findings represents not only the first clear evidence as to the function of CBS domains, but also the first description of the biochemical defect in several human hereditary diseases where mutations occur in these domains.

Our results strongly support the idea that the four tandem pairs of CBS domains in the γ subunits of AMPK (the only cases where four, rather than two, tandem CBS domains occur) provide two allosteric binding sites for AMP and ATP in the heterotrimeric complex, one or both of which are therefore key targets for development of drugs aimed at obesity and Type 2 diabetes. The evidence in favour of this may be summarised as follows:
1) While the N-terminal and C-terminal pairs of CBS domains from the γ2 subunit both bound one molecule of AMP when expressed in and purified from bacteria, the construct containing all four domains bound two molecules of AMP.
2) Mutations that caused an increase in the B_{0.5} for binding of AMP to the CBS domains of γ2 also caused an increase in the A_{0.5} for activation of recombinant α1β1γ2 complexes, with the same order of potency, i.e. B_{0.5}/A_{0.5} for wild type = *Lins* < R302Q < H383R < T400N < R531G.
3) ATP also bound to the bacterially expressed CBS domains in a mutually exclusive manner with AMP, albeit with somewhat lower affinity. This is consistent with the fact that high concentrations of ATP inhibit allosteric activation of AMPK in a manner that appears to be competitive with AMP (11). Although mutually exclusive binding to the bacterially expressed CBS domains does not prove that AMP and ATP bind to the same site, this is the simplest explanation that is also consistent with our findings that the γ2 mutations reduced the affinity for ATP in the same order in which they reduced the affinity for AMP (wild type ≈ Lins < R302Q < H383R < T400N < R531G). The effects of ATP on the activity of the heterotrimeric complex are more difficult to study in a quantitative manner, because the nucleotide binds to the catalytic as well as to the allosteric site. Nevertheless, it is clear that the affinity for ATP at the allosteric site is lower than that for AMP (11).

Our studies on AMP activation of heterotrimeric AMPK complexes are in good agreement with those of Daniel and Carling (36) who found that mutations associated with WPWS exhibited defective activation by AMP, with the effect on A_{0.5} increasing in the order WT ≈ *Lins* = R302Q < H383R (the T400N mutant was not studied). Like ourselves, Daniel and Carling found that the maximal stimulation by AMP was reduced by the R302Q mutation, while with the R531 G mutant the stimulation by AMP was abolished and the basal activity measured in the absence of AMP was slightly higher. They suggested that the latter effect might be because the inhibitory effect of ATP might also be reduced with this mutant. They did not directly measure the binding of ATP to the wild type or any of the mutants, but this hypothesis is supported by our findings that the affinity of the R531 G mutant for ATP, when expressed in the context of either CBS1-2 or CBS1-4 (Table 1), was greatly reduced.

Neither our results nor those of Daniel and Carling (36) support two previous claims (23, 35) that WPWS mutations make AMPK complexes constitutively active. When the cells were harvested by "rapid lysis" (i.e. by detergent lysis *in situ* on the culture dish, which we believe much better preserves the *in vivo* phosphorylation state of AMPK) and the kinase assayed in the presence of AMP, none of the mutants exhibited greater activity than the wild type (Fig. 4C). When the cells were harvested by the more conventional "slow lysis" procedure (which causes activation of AMPK due to hypoxia, glucose deprivation and/or mechanical stress), the mutants that exhibited defective activation by AMP *in vitro* (R302Q, H383R, T400N, R531G), were all activated to a lower extent than the wild type. Of the two previous claims that the mutations caused constitutive activation of AMPK, both relied on indirect approaches. One study involved making a mutation equivalent to R302Q in the γ1 rather than the γ2 isoform, and either expressing it by transient transfection in COS7 cells with α1 and β1, or in PS120 cells by stable transfection on its own (35). However, naturally occurring mutations at this position in γ1 have not been reported, and it is not known whether they would cause disease. The other study involved making mutations equivalent to T400N and N488I in the yeast γ subunit homologue, Snf4, and analysing two-hybrid interactions with Snf1 (the a homologue) as a surrogate measure of kinase activity (23). In fact, while both mutations did appear to cause small (2-fold) increases in the Snf1:Snf4 interaction under basal conditions, removal of glucose from the medium (known to activate the SNF1 complex (37)) appeared to cause further large increases in interaction with both of the mutants as well as with the wild type. The use of the term "constitutively active" to describe these Snf4 mutants (23) is therefore misleading. Our results and those of Daniel and Carling (36) suggest that while the R531G mutant has a slightly elevated basal activity (due perhaps to reduced binding of the inhibitor, ATP), the major effect of the mutations is to cause reduced activation of AMPK in response to stress.

Our results with IMPDH2 and CLC2 suggest that sensing of cellular energy status by binding of adenine nucleotides may be a general function of CBS domain pairs, rather than being a function restricted to AMPK. The CBS domain pairs from IMPDH2 also bound AMP and GMP *in vitro* in addition to ATP, but only the latter bound at physiologically relevant concentrations. IMP dehydrogenases catalyse the first step in purine nucleotide biosynthesis that is committed to synthesis of GMP rather than AMP. They are subject to feedback inhibition by GMP, but GMP did not bind with high affinity to the CBS domains from IMPDH2. Since inhibition by GMP is competitive with the substrate, IMP (38), it is more likely that the feedback inhibition is due to binding of GMP to the IMP-binding site in the catalytic domain. It is interesting that, while ATP bound to the isolated CBS domain pair from IMPDH2 with a Kd in the low µM range (54 µM) with no evidence for interaction between sites, it bound to full length enzyme with positive co-operativity and a B_{0.5} (770 µM) that was 14-fold higher and much closer to the physiological range of ATP concentrations. The crystal structures of mammalian and bacterial IMPDH show that the enzyme is a tetramer with the CBS domain pairs on the outside, with the subunit contacts being made entirely by the catalytic domains (39, 40). Our results suggest that the CBS domain pair in the tetramer is constrained in a conformation that has a lower affinity for ATP than the isolated CBS domains, but that binding of ATP to the first site causes a conformational change that is somehow transmitted across the subunit interface to increase the affinity of the remaining CBS domain pairs. We were unable to find any previous reports in the literature that IMPDH was activated by ATP, but our results now show for the first time that the nucleotide increases the Vmax of the enzyme by >4-fold. The reasonably close correspondence between the B_{0.5} for ATP binding (770 µM) and the A_{0.5} for ATP activation (440 µM) of the tetramer, and the finding that the R224P mutation in the second CBS domain abolishes both binding of ATP and activation by ATP, provides strong evidence that the allosteric activation is due to binding of the nucleotide to the CBS domains. We propose that this mechanism ensures that the synthesis of guanine nucleotides only occurs when the supply of ATP is sufficient, a mechanism that accompanies feedback inhibition by GMP. This is highly analogous with the regulation of the key enzyme of pyrimidine nucleotide synthesis, aspartate transcarbamylase, in bacteria, which is inhibited by the end product CTP while being activated by ATP (41). However, the ATP binding domains on aspartate transcarbamylase are not related to CBS domains.

Our results also suggest that the natural ligand for the CBS domains of CLC chloride channels is ATP, and that a mutation in CLC2 (G715E) associated with idiopathic generalized epilepsy (29), and a second mutation (G826D) equivalent to a mutation in CLC1 associated with congenital myotonia (28), both lead to a severe defect in ATP binding. While our work was in progress, Vanoye and George (42) reported using patch clamp analysis that the human CLC4 channel only supported a chloride current when incubated on the internal side of the membrane with ATP or a non-hydrolyzable ATP analogue. The CBS domain pairs of the CLC family occur at the C-terminus, which is predicted to lie on the cytoplasmic side of the plasma membrane. Our results and those of Vanoye and George suggest that binding of ATP to the CBS domains of the CLC chloride channels is necessary before the channels will open.

We therefore propose that, in most cases, tandem pairs of CBS domains form sensors of cellular energy status that act by binding AMP and/or ATP. An exception to this is appears to be cystathionine β-synthase itself (from which the acronym CBS is derived), which catalyses the first step in cysteine synthesis and is allosterically activated by Sadenosyl methionine (SAM). Its substrate, homocysteine, is an intermediate in the "activated methyl cycle" in which SAM (an important donor of methyl groups during biosynthesis) is regenerated from S-adenosyl homocysteine. A low activity of cystathionine β-synthase would promote recycling of homocysteine into SAM, whereas activation of the enzyme by high concentrations of SAM would favour the removal of homocysteine from the cycle and its conversion to cysteine instead. Intriguingly, point mutations in the CBS domains of cystathionine β-synthase, or premature termination or proteolysis that removes them, result in enzyme that no longer responds to SAM (16, 43, 18). These results suggested, but did not prove, that the CBS domains might represent the binding site for SAM. Our present results now provide strong support for this hypothesis, since the isolated CBS domains bind one molecule of SAM with a Kd of 34 µM, while the D444N mutation that causes homocystinuria drastically reduces the affinity for SAM (Fig. 7). These results are consistent with those of Kluijtmans et al (16), who found that wild type cystathionine β-synthase was activated by SAM with a half-maximal effect at about 20 µM, while the D444N mutant was not significantly activated by concentrations of SAM up to 300 µM. In the case of cystathionine β-synthase, the CBS domains appear to have been adapted to bind not an adenine nucleotide, but a related adenosine-containing compound, Sadenosyl methionine. In fact the second, C-terminal CBS domain in cystathionine β-synthase is rather poorly conserved ((15); see also the PFAM database (44), entry #PF00571). In addition, a truncation that removes the N-terminal 70 residues of cystathionine β-synthase, which contains the heme-binding region, results in enzyme that retains 20% of wild type activity but is no longer activated by SAM (45). One possibility is that the CBS domains of cystathionine β-synthase bind the adenosyl moiety of SAM, whereas the N-terminal heme domain is involved in binding of the methionine moiety, which is not present in the ligands that bind to other CBS domain pairs.

Although our present results provide strong support for the idea that the CBS domains of the γ subunits of AMPK provide the allosteric binding sites for AMP and ATP, a number of puzzling features remain to be addressed in future studies. Firstly, the A_{0.5} values for activation of wild type and mutant versions of the α1 β1γ2 complex by AMP were generally around 10-fold lower than the B_{0.5} values for binding of AMP to the equivalent CBS1-4 constructs. Secondly, the B_{0.5.} for binding of AMP to the wild type CBS1-4 constructs was different for the three γ subunit isoforms (γ1, 20 µM; γ2, 53 µM; γ3, 125 µM), but the recombinant α1β1γ1, α1β1γ2 and α1β1γ3 complexes were all activated by similar, lower concentrations of AMP (A_{0.5} = 2-13 µM), and differed instead in their degree of stimulation by AMP (Fig. 4B). The latter data are in good agreement with previous results obtained with native α1β1γ1, α1β1γ2 and α1β1γ3 complexes prepared by immunoprecipitation from rat liver or brain extracts (14). What is the explanation for these differences? One possibility is that the binding of the a subunit to the γ subunit might alter the conformation of the latter to change the affinity for the regulatory nucleotide. Alternatively, the α subunit might be providing additional interactions with the ligand that increases the affinity in the heterotrimeric αβγ complex. This second possibility is supported by our previous findings that, whereas a photolabile analogue of AMP (8-azido-AMP) labels the γ subunit (14), a reactive analogue of ATP, i.e. p-fluorosulfonyl benzoyl adenosine (where the reactive group occupies a position equivalent to the γ phosphate of ATP) clearly binds at the allosteric site but labels only the α subunit (46). This suggests that when the inhibitory ligand, ATP, is bound to the CBS domains of the γ subunit it also contacts the α subunit.

Another unexpected finding of our study was that the CBS domains of γ2 contain two binding sites for both AMP and ATP (although given that there are four, rather than two, tandem CBS domains this makes sense with hindsight). There is an interesting parallel here with the regulatory subunits of cyclic AMP-dependent protein kinase, which contain two tandem binding sites for cyclic AMP, although these are not related in sequence to CBS domains. A current model for activation of cyclic AMP-dependent protein kinase is that cyclic AMP binds initially to the C-terminal site (site B), and only then does site A become accessible, although it is binding to site A that causes activation (47). Further work is required to determine whether a similar mechanism operates in the case of AMPK. The finding that mutations in CBS1 (R302Q), CBS2 (H383R, T400N) and CBS4 (R531 G) all affect AMP activation (Fig. 4A) suggests that AMP must bind to both sites for activation to occur. However, a sigmoidal activation of AMPK by AMP (which might be expected if occupancy of both binding sites was necessary for activation) has not been observed by ourselves, or reported by others to our knowledge. This may be because quantitative measurements of allosteric activation of AMPK by AMP are more difficult to perform and interpret than measurements of nucleotide binding to the isolated CBS domains, for the following reasons: (i) the kinase complex has a significant basal activity, so that there is a higher background; (ii) AMP and ATP are simultaneously present, and bind antagonistically not only at the two allosteric sites but also at the catalytic site. It is therefore conceivable that a sigmoidal dependence of activation on AMP concentration could have been missed for technical reasons. Structural models of AMPK domains and complexes, obtained in the presence and absence of ligands, will probably be required to fully explain these subtleties in the regulation of the kinase. However, if activation by AMP is indeed co-operative this would be a further mechanism to increase the sensitivity of the AMPK system to small changes in the concentration of the activating nucleotide (10).

Finally, one interesting feature of the pathogenic mutations in CBS domains is that they tend to occur in the same positions. Thus, the R302Q (21), H383R (22) and R531G (20) mutations in CBS1, CBS2 and CBS4 of γ2 that cause human WPWS all align (±1 residue) with the D444N mutation (16) in cystathionine β-synthase causing homocystinuria, as well as an R200Q mutation in CBS1 of the γ3 isoform of pig AMPK that causes abnormally high muscle glycogen content (19). Similarly, the T400N (23) mutation in CBS2 of γ2 causing WPWS aligns (±1 residue) with the R224P mutation (27) in CBS2 of IMPDH1 that causes retinitis pigmentosa. These "hot spots" for pathogenic mutations are likely to be directly involved in binding of the adenosine-containing ligand. A model for the N-terminal domain pair in the γ2 subunit of AMPK, based on the atomic co-ordinates of a bacterial IMP dehydrogenase (39), is shown in Fig. 8. Two four stranded β sheets, comprising three strands from one domain and one from the other, form a deep hydrophobic cleft between the two domains that is of suitable dimensions to accommodate an adenosine moiety. Many of the residues that are mutated in disease states have basic, positively charged side chains (e.g. Arg302 and His383 in γ2, and Arg224 in IMPDH2, which aligns with His401 in γ2), and are predicted to lie around the mouth of this cleft (Fig. 8). In the CBS domain pairs that bind AMP and/or ATP these residues form a positively charged patch at the mouth of the cleft, which is likely to bind the α phosphate moieties of the adenine nucleotides. Other residues that are mutated (Thr400 in γ2, and Gly828 in CLC2, aligning with Tyr397 in γ2) are predicted to lie within the cleft itself, where they may form interactions with the ribose or adenine moieties. Another notable feature is that the basic residues that form the basic, positively charged patch at the mouth of the cleft are either uncharged or acidic in cystathionine β-synthase. The D444N mutation in this protein, which causes homocystinuria, would neutralize a negative charge that occurs in the equivalent position to Arg302 in γ2. Intriguingly, the ligand for cystathionine β-synthase. i.e. S-adenosyl methionine, has a positively charged sulfur atom in approximately the same position as the negatively charged phosphate of AMP, and it is likely that Asp444 forms an electrostatic interaction with this sulfur atom.

Our new findings represent a novel method to screen for compounds that bind to the allosteric sites on AMPK, and by expressing CBS1-2, CBS3-4 or CBS1-4 separately we also introduce the ability to screen for compounds that bind at the N-terminal site, the C-terminal site, or both. The methods developed here should be equally applicable to the CBS domains of the γ3 isoform. Thus it would be possible to generate screens for compounds that might be selective activators or inhibitors of γ1, γ2 or γ3. Our method does not distinguish between activators or inhibitors, but that could be easily checked with any lead compounds using purified αβγ complexes. Finally, the methods described herein allow the production of functional AMP/ATP binding domains in quantities of tens of milligrams. Thus it should be possible to crystallize the domains in the presence or absence of ligands, determine the atomic structures of the complexes, and use this to optimise the ligand structure to obtain the most potent activators.

**Table 1: Binding parameters for AMP and ATP measured with various constructs of AMPK, IMPDH2 and CLC2.**

| Protein | Construct | n⁽¹⁾ | Kd⁽²⁾ or B_{0.5} AMP (µM) | *h*⁽³⁾ AMP | Kd⁽²⁾ or B_{0.5} ATP (µM) | *h*⁽³⁾ ATP |
|---|---|---|---|---|---|---|
| AMPK-γ2 | CBS 1-2 (WT) | 1 | 53 ± 5 | - | 175 ± 20 | - |
| | CBS1-2 (R302Q) | 1 | 303 ± 42 | - | 468 ± 86 | - |
| | CBS1-2 (L*ins*) | 1 | 63 ± 7 | - | 202 ± 22 | - |
| | CBS1-2 (H383R) | 1 | 1500 ± 210 | - | 1570 ± 140 | - |
| | CBS1-2 (T400N) | 1 | 556 ± 56 | - | 952 ± 55 | - |
| | CBS3-4 (WT) | 1 | 104 ± 10 | - | 403 ± 16 | - |
| | CBS3-4 (R531G) | 1 | 1570 ± 110 | - | 2050 ± 150 | - |
| | CBS1-4 (WT) | 2 | 61 ± 2 | 2.2 ± 0.1 | 257 ± 14 | 2.2 ± 0.2 |
| | CBS1-4 (R302Q) | 2 | 158 ± 9 | 1.9 ± 0.2 | 193 ± 25 | 1.3 ± 0.2 |
| | CBS 1-4 (L*ins*) | 2 | 130 ± 4 | 1.8 ± 0.1 | 335 ± 28 | 1.8 ± 0.2 |
| | CBS 1-4 (H383R) | 2 | 725 ± 36 | 1.8 ± 0.2 | 1470 ± 55 | 2.4 ± 0.2 |
| | CBS 1-4 (T400N) | 2 | 1360 ± 47 | 1.8 ± 0.1 | 2000 ± 160 | 1.7 ± 0.2 |
| | CBS 1-4 (R531G) | 2 | 2800 ± 220 | 1.6 ± 0.2 | 2550 ± 230 | 2.7 ± 0.5 |
| AMPK-γ1 | CBS1-4 | 2 | 20 ± 1 | 1.6 ± 0.2 | 119 ± 7 | 1.9 ± 0.2 |
| AMPK-γ3 | CBS1-4 | 2 | 125 ± 11 | 2.2 ± 0.1 | 989 ± 58 | 1.5 ± 0.1 |
| IMPDH2 | CBS 1-2 (WT) | 1 | 437 ± 53 | - | 54 ± 6 | - |
| | CBS1-2 (R224P) | 1 | - | - | 450 ± 36 | - |
| | Tetramer (WT) | 4 | - | - | 770 ± 50 | 1.7 ± 0.2 |
| | Tetramer (R224P) | 4 | - | - | 64000 ± 5000 | 0.9 ± 0.1 |
| CLC2 | CBS1-2 (WT) | 1 | 1060 ± 80 | - | | |
| | CBS1-2 (G715E) | 1 | 10400 ± 1300 | - | | |
| | | 1 | 14300 ± 550 | - | | |
| CBS | CBS1-2 (WT) | 1 | 34 ± 2⁽⁴⁾ | | | |
| | D444N | 1 | 510 ± 70⁽⁴⁾ | | | |

| | | | | | | |
|---|---|---|---|---|---|---|
| Notes: (1) n = number of binding sites assumed per protein molecule; (2) where a single binding site is assumed, the data was fitted to a simple binding equation (Y = L/(Kd+L)) where Y = =fractional saturation and L = ligand concentration, and the value given in this column is Kd; where>1 binding site is assumed, the data was fitted to the Hill equation (Y = L^{h}/(B_{0.5}^{h}+L^{h})) and the value given in this column is the B_{0.5}, i.e. the concentration of ligand giving half-maximal binding; (3) *h* = Hill coefficient (4) these are Kd values for S-adenosyl methionine, not AMP | | | | | | |

### References

[1] Hardie, D.G., Carling, D. and Carlson, M. (1998) Ann. Rev. Biochem. 67, 821-855. "The AMP-activated/SNF1 protein kinase subfamily: metabolic sensors of the eukaryotic cell?"
[2] Hardie, D.G. and Hawley, S.A. (2001) BioEssays 23, 1112-1119. "AMP-activated protein kinase: the energy charge hypothesis revisited".
[3] Hardie, D.G. and Carling, D. (2002) J. Physiol., in press. "The role of AMP-activated protein kinase in muscle metabolism".
[4]Winder, W.W. and Hardie, D.G. (1999) Am. J. Physiol. 277, E1-E10. "The AMP-activated protein kinase, a metabolic master switch: possible roles in Type 2 diabetes".
[5] Zhou, G. et al. (2001) J. Clin. Invest. 108, 1167-1174. "Role of AMP-activated protein kinase in mechanism of metformin action".
[6] Fryer, L.G., Parbu-Patel, A. and Carling, D. (2002) J. Biol. Chem., in press. "The anti-diabetic drugs rosiglitazone and metformin stimulate AMP-activated protein kinase through distinct pathways".
[7] Hawley, S.A., Gadalla, A.E., Olsen, G.S. and Hardie, D.G. (2002) Diabetes 51, 2420-2425. "The anti-diabetic drug metformin activates the AMP-activated protein kinase cascade via an adenine nucleotide-independent mechanism".
[8] Minokoshi, Y., Kim, Y.B., Peroni, O.D., Fryer, L.G., Muller, C., Carling, D. and Kahn, B.B. (2002) Nature 415, 339-343. "Leptin stimulates fatty-acid oxidation by activating AMP-activated protein kinase".
[9] Kay, J.P., Alemzadeh, R., Langley, G., D'Angelo, L., Smith, P. and Holshouser, S. (2001) Metabolism 50, 1457-1461. "Beneficial effects of metformin in normoglycemic morbidly obese adolescents".
[10] Hardie, D.G., Salt, I.P., Hawley, S.A. and Davies, S.P. (1999) Biochem. J. 338, 717-722. "AMP-activated protein kinase: an ultrasensitive system for monitoring cellular energy charge".
[11] Corton, J.M., Gillespie, J.G., Hawley, S.A. and Hardie, D.G. (1995) Eur. J. Biochem. 229, 558-565. "5-Aminoimidazole-4-carboxamide ribonucleoside: a specific method for activating AMP-activated protein kinase in intact cells?"
[12] Hawley, S.A., Selbert, M.A., Goldstein, E.G., Edelman, A.M., Carling, D. and Hardie, D.G. (1995) J. Biol. Chem. 270, 27186-27191. "5'-AMP activates the AMP-activated protein kinase cascade, and Ca2+/calmodulin the calmodulin-dependent protein kinase cascade, via three independent mechanisms".
[13] Davies, S.P., Helps, N.R., Cohen, P.T.W. and Hardie, D.G. (1995) FEBS Lett. 377, 421-425. "5'-AMP inhibits dephosphorylation, as well as promoting phosphorylation, of the AMP-activated protein kinase. Studies using bacterially expressed human protein phosphatase-2Ca and native bovine protein phosphatase-2AC".
[14] Cheung, P.C.F., Salt, I.P., Davies, S.P., Hardie, D.G. and Carling, D. (2000) Biochem. J. 346, 659-669. "Characterization of AMP-activated protein kinase γ subunit isoforms and their role in AMP binding".
[15] Bateman, A. (1997) Trends Biochem. Sci. 22, 12-13. "The structure of a domain common to archaebacteria and the homocystinuria disease protein".
[16] Kluijtmans, L.A., Boers, G.H., Stevens, E.M., Renier, W.O., Kraus, J.P., Trijbels, F.J., van den Heuvel, L.P. and Blom, H.J. (1996) J. Clin. Invest. 98, 285-9. "Defective cystathionine b-synthase regulation by S-adenosylmethionine in a partially pyridoxine responsive homocystinuria patient".
[17] Shan, X., Dunbrack, R.L., Jr., Christopher, S.A. and Kruger, W.D. (2001) Hum. Mol. Genet. 10, 635-43. "Mutations in the regulatory domain of cystathionine b-synthase can functionally suppress patient-derived mutations in cis".
[18] Kery, V., Poneleit, L. and Kraus, J.P. (1998) Arch. Biochem. Biophys. 355, 222-232. "Trypsin cleavage of human cystathionine b-synthase into an evolutionarily conserved active core: structural and functional consequences".
[19] Milan, D. et al. (2000) Science 288, 1248-1251. "A mutation in PRKAG3 associated with excess glycogen content in pig skeletal muscle".
[20] Gollob, M.H., Seger, J.J., Gollob, T.N., Tapscott, T., Gonzales, 0., Bachinski, L. and Roberts, R. (2001) Circulation 104, 3030-3033. "Novel PRKAG2 mutation responsible for the genetic syndrome of ventricular preexcitation and conduction system disease with childhood onset and absence of cardiac hypertrophy".
[21] Gollob, M.H. et al. (2001) New Eng. J. Med. 344, 1823-1831. "Identification of a gene responsible for familial Wolff-Parkinson-White syndrome".
[22] Blair, E. et al. (2001) Hum. Mol. Genet. 10, 1215-1220. "Mutations in the gamma-2 subunit of AMP-activated protein kinase cause familial hypertrophic cardiomyopathy: evidence for the central role of energy compromise in disease pathogenesis".
[23] Arad, M. et al. (2002) J. Clin. Invest. 109, 357-362. "Constitutively active AMP kinase mutations cause glycogen storage disease mimicking hypertrophic cardiomyopathy".
[24] Hawley, S.A., Davison, M., Woods, A., Davies, S.P., Beri, R.K., Carling, D. and Hardie, D.G. (1996) J. Biol. Chem. 271, 27879-27887. "Characterization of the AMP-activated protein kinase kinase from rat liver, and identification of threonine-172 as the major site at which it phosphorylates and activates AMP-activated protein kinase".
**[25]** Woods, A., Salt, I., Scott, J., Hardie, D.G. and Carling, D. (1996) FEBS Lett. 397, 347-351. "The a1 and a2 isoforms of the AMP-activated protein kinase have similar activities in rat liver but exhibit differences in substrate specificity in vitro".
[26]. Bowne, S.J., Sullivan, L.S., Blanton, S.H., Cepko, C.L., Blackshaw, S., Birch, D.G., Hughbanks-Wheaton, D., Heckenlively, J.R., and Daiger, S.P. 2002. Mutations in the inosine monophosphate dehydrogenase 1 gene (IMPDH1) cause the RP10 form of autosomal dominant retinitis pigmentosa. Hum. Mol. Genet. 11:559-568.
[27] Kennan, A., Aherne, A., Palfi, A., Humphries, M., McKee, A., Stitt, A., Simpson, D.A., Demtroder, K., Orntoft, T., Ayuso, C., et al. 2002. Identification of an IMPDH1 mutation in autosomal dominant retinitis pigmentosa (RP10) revealed following comparative microarray analysis of transcripts derived from retinas of wild-type and Rho(-/-) mice. Hum. Mol. Genet. 11:547-557.
[28] Pusch, M. 2002. Myotonia caused by mutations in the muscle chloride channel gene CLCN1. Hum. Mutat. 19:423-434.
**[29]** Haug, K., Warnstedt, M., Alekov, A.K., Sander, T., Ramirez, A., Poser, B., Maljevic, S., Hebeisen, S., Kubisch, C., Rebstock, J., et al. 2003. Mutations in CLCN2 encoding a voltage-gated chloride channel are associated with idiopathic generalized epilepsies. Nat. Genet. 33:527-532.
[30] Lloyd, S.E., Gunther, W., Pearce, S.H., Thomson, A., Bianchi, M.L., Bosio, M., Craig, I.W., Fisher, S.E., Scheinman, S.J., Wrong, O., et al. 1997. Characterisation of renal chloride channel, CLCN5, mutations in hypercalciuric nephrolithiasis (kidney stones) disorders. Hum. Mol. Genet. 6:1233-1239.
**[31]** Konrad, M., Vollmer, M., Lemmink, H.H., van den Heuvel, L.P., Jeck, N., Vargas-Poussou, R., Lakings, A., Ruf, R., Deschenes, G., Antignac, C., et al. 2000. Mutations in the chloride channel gene CLCNKB as a cause of classic Bartter syndrome. J. Am. Soc. Nephrol. 11:1449-1459.
[32]. Woods, A., Cheung, P.C.F., Smith, F.C., Davison, M.D., Scott, J., Beri, R.K., and Carling, D. 1996. Characterization of AMP-activated protein kinase β and γ subunits: assembly of the heterotrimeric complex in vitro. J. Biol. Chem. 271:10282-10290.
[33]. Hardie, D.G., Salt, I.P., and Davies, S.P. 2000. Analysis of the role of the AMPactivated protein kinase in the response to cellular stress. Methods Mol. Biol. 99:63-75.
[34]. Stein, S.C., Woods, A., Jones, N.A., Davison, M.D., and Carling, D. 2000. Theregulation of AMP-activated protein kinase by phosphorylation. Biochem. J. 345:437-44335.
[35] Hamilton, S.R., Stapleton, D., O'Donnell, J.B., Kung, J.T., Dalal, S.R., Kemp, B.E., and Witters, L.A. 2001. An activating mutation in the γ1 subunit of the AMP-activated protein kinase. FEBS Lett. 500:163-168.
[36].Daniel, T.D., and Carling, D. 2002. Functional analysis of mutations in the γ2 subunit of AMP-activated protein kinase associated with cardiac hypertrophy and Wolff-Parkinson-White syndrome. J. Biol. Chem. 277:51017-51024.
[37]. Wilson, W.A., Hawley, S.A., and Hardie, D.G. 1996. The mechanism of glucose repression/derepression in yeast: SNF1 protein kinase is activated by phosphorylation under derepressing conditions, and this correlates with a high AMP:ATP ratio. Current Biol. 6:1426-1434.
[38]. Gilbert, H.J., Lowe, C.R., and Drabble, W.T. 1979. Inosine 5'-monophosphate dehydrogenase of Escherichia coli. Purification by affinity chromatography, subunit structure and inhibition by guanosine 5'-monophosphate. Biochem. J. 183:451-494.
[39]. Zhang, R., Evans, G., Rotella, F.J., Westbrook, E.M., Beno, D., Huberman, E., Joachimiak, A., and Collart, F.R. 1999. Characteristics and crystal structure of bacterial inosine-5'- monophosphate dehydrogenase. Biochemistry 38:4691-4700.
[40]. Sintchak, M.D., Fleming, M.A., Futer, O., Raybuck, S.A., Chambers, S.P., Caron, P.R., Murcko, M.A., and Wilson, K.P. 1996. Structure and mechanism of inosine monophosphate dehydrogenase in complex with the immunosuppressant mycophenolic acid. Cell 85:921-930.
**[41]**. Fetler, L., and Vachette, P. 2001. The allosteric activator Mg-ATP modifies the quaternary structure of the R-state of Escherichia coli aspartate transcarbamylase without altering the T<-->R equilibrium. J. Mol. Biol. 309:817-832.
[42] Vanoye, C.G., and George, A.G., Jr. 2002. Functional characterization of recombinant human CIC-4 chloride channels in cultured mammalian cells. J. Physiol. 539:373-383.
[43]. Shan, X., Dunbrack, R.L., Jr., Christopher, S.A., and Kruger, W.D. 2001. Mutations in the regulatory domain of cystathionine β-synthase can functionally suppress patientderived mutations in cis. Hum. Mol. Genet. 10:635-643.
[44]. Bateman, A., Birney, E., Cerruti, L., Durbin, R., Etwiller, L., Eddy, S.R., Griffiths-Jones, S., Howe, K.L., Marshall, M., and Sonnhammer, E.L. 2002. The Pfam protein families database. Nucleic Acids Res. 30:276-280.
[45]. Oliveriusova, J., Kery, V., Maclean, K.N., and Kraus, J.P. 2002. Deletion mutagenesis of human cystathionine beta-synthase. Impact on activity, oligomeric status, and Sadenosylmethionine regulation. J. Biol. Chem. 277:48386-48394.
[46]. Carling, D., Clarke, P.R., Zammit, V.A., and Hardie, D.G. 1989. Purification and characterization of the AMP-activated protein kinase. Copurification of acetyl-CoA carboxylase kinase and 3-hydroxy-3-methylglutaryl-CoA reductase kinase activities. Eur. J. Biochem. 186:129-136.
[47]. Su, Y., Dostmann, W.R., Herberg, F.W., Durick, K., Xuong, N.H., Ten Eyck, L., Taylor, S.S., and Varughese, K.I. 1995. Regulatory subunit of protein kinase A: structure of deletion mutant with cAMP binding domains. Science 269:807-813.

## Claims

1. A method for identifying a substance capable of binding to the γ subunit of AMP-activated protein kinase (AMPK), or a fragment, derivative, homologue or mutant thereof, which method comprises:
a) providing a γ subunit polypeptide or a fragment, homologue, derivative or mutant thereof, wherein said fragment is a portion of a γ subunit, which is capable of binding 5'-AMP and/or ATP; a homologue is a variant of a γ subunit AMPK from a different species; a mutant comprises a deletion, substitution, insertion or inversion of (an) amino acid(s) in said γ subunit AMPK sequence but which mutant AMPK shows the recognised activity of wild-type AMPK; and a derivative comprises said γ subunit, homologue, or mutant as defined above, fused to another polypeptide;
b) contacting the γ subunit polypeptide, a fragment, homologue, derivative or mutant thereof, with a test substance under conditions that would permit 5'-AMP and/or ATP to bind with the γ subunit polypeptide; and
c) determining whether said test substance has bound to the γ subunit polypeptide, a fragment, homologue, derivative or mutant thereof

2. The method according to claim 1 further comprising the step of administering a substance, which has been determined as being able to bind the γ subunit polypeptide of AMPK, to AMPK, in order to determine any modulatory effect of the substance on AMPK activity.

3. The method according to claim 2 for identifying an agonist of AMPK.

4. The method according to claim 2 for identifying an antagonist of AMPK.

5. The method according to any preceding claim wherein the γ subunit is the γ1, γ2 and/or γ3 subunit of AMPK.

6. The method according to any preceding claim wherein a fragment of the γ subunit is employed.

7. The method according to claim 6 wherein the fragment comprises at least one CBS domain.

8. The method according to claim 7 wherein the fragment comprises CBS domains 1 and 2, 3 and 4 or 1 to 4.

9. The method according to any preceding claim wherein the γ subunit or fragment is a γ2 subunit or fragment and comprises a mutation(s), R302Q, H383R, T400N, *Lins* and/or R531G.

10. The method according to any preceding claim wherein the γ subunit is AMPK is in the form of a fusion protein.

11. The method according to any preceding claim wherein the γ subunit, fragment, derivative, homologue or mutant thereof has been recombinantly produced.

12. The method according to any preceding claim further comprising identifying if the substance is capable of binding to another domain of AMPK.

13. The method according to claim 12 wherein the domain of AMPK is the kinase domain.

14. The method according to any preceding claim wherein the test agent is chosen to selectively bind to the γ subunit domain in preference to any other domain.

15. The method according to any preceding claim wherein the test agent is chosen to selectively bind to the γ subunit of AMPK over a CBS domain(s) from another protein.

16. The method according to claim 15 wherein the other protein is IMP dehydrogenase, CLC chloride channel proteins and/or cystathionine β-synthase.

17. A method of identifying a substance capable of binding to a CBS domain of a protein possessing such a CBS domain or domains, or a fragment, derivative, homologue or mutant thereof, which method comprises:
a) providing a CBS domain subunit polypeptide, or a fragment, homologue, derivative or mutant thereof wherein said fragment is a portion of a CBS subunit, which is capable of binding 5'-AMP and/or ATP; a homologue is a variant of a CBS subunit AMPK from a different species; a mutant comprises a deletion, substitution, insertion or inversion of (an) amino acid(s) in said CBS subunit AMPK sequence but which mutant AMPK shows the recognised activity of wild-type AMPK; and a derivative comprises said γ subunit, homologue, or mutant as defined above, fused to another polypeptide;
b) contacting the CBS domain subunit polypeptide, or a fragment, homologue, derivative or mutant thereof, with a test substance under conditions that would permit 5'-AMP and/or ATP to bind with the CBS domain subunit polypeptide; and
c) determining whether said test substance has bound to the CBS domain subunit polypeptide.

## Patentansprüche

1. Ein Verfahren zur Identifizierung einer Substanz, welche fähig ist zur Bindung an die γ-Untereinheit von AMP-aktivierter Proteinkinase (AMPK) oder ein Fragment, Derivat, Homolog oder eine Mutante von dieser, wobei das Verfahren umfasst:
a) Bereitstellen eines γ-Untereinheit-Polypeptids oder eines Fragmentes, Homologs, Derivates oder einer Mutante von diesem, wobei das Fragment ein Teil einer γ-Untereinheit ist, welcher fähig ist zur Bindung von 5'-AMP und/oder ATP; ein Homolog ist eine Variante von einer γ-Untereinheit der AMPK von einer anderen Spezies; eine Mutante umfasst eine Deletion, Substitution, Insertion oder Inversion von (einer) Aminosäure(n) in der Sequenz der γ-Untereinheit von AMPK, aber deren Mutanten-AMPK zeigt die erkannte Aktivität der Wildtyp-AMPK; und ein Derivat umfasst die γ-Untereinheit, ein Homolog oder eine Mutante, wie oben definiert, fusioniert an einem anderen Polypeptid;
b) in Kontakt bringen des γ-Untereinheit-Polypeptids, eines Fragmentes, eines Homologs, eines Derivates oder einer Mutante davon mit einer Testsubstanz unter Bedingungen, welche 5'-AMP und/oder ATP eine Bindung mit dem γ-Untereinheit-Polypeptid ermöglichen würden; und
c) Bestimmung, ob die Testsubstanz an das γ-Untereinheit-Polypeptid gebunden hat, an ein Fragment, Homolog, Derivat oder eine Mutante davon.

2. Das Verfahren nach Anspruch 1, weiterhin umfassend den Schritt der Verabreichung einer Substanz, bei welcher die Fähigkeit nachgewiesen worden ist, das γ-Untereinheit-Polypeptid von AMPK zu binden, zu AMPK, um einen modulatorischen Effekt der Substanz auf die AMPK-Aktivität zu bestimmen.

3. Das Verfahren nach Anspruch 2 zur Identifizierung eines Agonisten von AMPK.

4. Das Verfahren nach Anspruch 2 zur Identifizierung eines Antagonisten von AMPK.

5. Das Verfahren nach einem der vorangehenden Ansprüche, wobei die γ-Untereinheit die γ1, γ2 und/oder γ3 -Untereinheit von AMPK ist.

6. Das Verfahren nach einem der vorangehenden Ansprüche, wobei ein Fragment der γ-Untereinheit verwendet wird.

7. Das Verfahren nach Anspruch 6, wobei das Fragment mindestens eine CBS-Domäne umfasst.

8. Das Verfahren nach Anspruch 7, wobei das Fragment die CBS-Domänen 1 und 2, 3 und 4 oder 1 bis 4 umfasst.

9. Das Verfahren nach einem der vorangehenden Ansprüche, wobei die γ-Untereinheit oder das Fragment eine γ2-Untereinheit oder deren Fragment ist und eine oder mehrere Mutationen, R302Q, H383R, T400N, L*ins* und/oder R531 G umfasst.

10. Das Verfahren nach einem der vorangehenden Ansprüche, wobei die γ-Untereinheit von AMPK in der Form eines Fusionsproteins vorliegt.

11. Das Verfahren nach einem der vorangehenden Ansprüche, wobei die γ-Untereinheit, das Fragment, Derivat, Homolog oder die Mutante davon rekombinant hergestellt worden ist.

12. Das Verfahren nach einem der vorangehenden Ansprüche, weiterhin umfassend die Feststellung, ob die Substanz fähig ist, an eine andere Domäne von AMPK zu binden.

13. Das Verfahren nach Anspruch 12, wobei die Domäne von AMPK die Kinase-Domäne ist.

14. Das Verfahren nach einem der vorangehenden Ansprüche, wobei das Testagens ausgewählt wird, um selektiv an die γ-Untereinheit-Domäne zu binden bevorzugt vor jeder anderen Domäne.

15. Das Verfahren nach einem der vorangehenden Ansprüche, wobei das Testagens ausgewählt wird, um selektiv an die γ-Untereinheit von AMPK zu binden über (eine) CBS-Domäne(n) eines anderen Proteins.

16. Das Verfahren nach Anspruch 15, wobei das andere Protein IMP-Dehydrogenase, CIC-Chloridkanal-Proteine und/oder Cystathionin-β-Synthase ist.

17. Ein Verfahren zur Identifizierung einer Substanz, welche fähig ist zur Bindung an eine CBS-Domäne eines Proteins, welches eine solche CBS-Domäne oder -Domänen besitzt, oder ein Fragment, Derivat, Homolog oder eine Mutante davon, wobei das Verfahren umfasst:
a) Bereitstellen eines Untereinheit-Polypeptids mit CBS-Domäne oder eines Fragmentes, Homologs, Derivates oder einer Mutante von diesem, wobei das Fragment ein Teil einer CBS-Untereinheit ist, welcher fähig ist zur Bindung von 5'-AMP und/oder ATP; ein Homolog ist eine Variante von einer CBS-Untereinheit der AMPK von einer anderen Spezies; eine Mutante umfasst eine Deletion, Substitution, Insertion oder Inversion von (einer) Aminosäure(n) in der Sequenz der CBS-Untereinheit von AMPK, aber deren Mutanten-AMPK zeigt die erkannte Aktivität der Wildtyp-AMPK, und ein Derivat umfasst die γ-Untereinheit, ein Homolog oder eine Mutante, wie oben definiert, fusioniert an ein anderes Polypeptid;
b) in Kontakt bringen der CBS-Domäne vom Untereinheit-Polypeptid, oder eines Fragmentes, Homologs, Derivates oder einer Mutante davon mit einer Testsubstanz unter Bedingungen, welche 5'-AMP und/oder ATP eine Bindung mit dem CBS-Untereinheit-Polypeptid ermöglichen würden; und
c) Bestimmung, ob die Testsubstanz an die CBS-Domäne des Untereinheit-Polypeptids gebunden hat.

## Revendications

1. Procédé d'identification d'une substance capable de se lier à la sous-unité γ de la protéine kinase activée par l'AMP (AMPK), ou un fragment, un dérivé, un homologue ou un mutant de celle-ci, lequel procédé comprend :
a) la fourniture d'un polypeptide d'une sous-unité γ ou un fragment, un homologue, un dérivé ou un mutant de celle-ci, dans lequel ledit fragment est une partie d'une sous-unité γ, qui est capable de se lier à du 5'-AMP et/ou de l'ATP; un homologue est un variant d'une sous-unité γ d'AMPK d'une espèce différente ; un mutant comprend une délétion, une substitution, une insertion ou une inversion d'un ou plusieurs acides aminés dans la séquence de ladite sous-unité γ de l'AMPK mais dont l'AMPK mutante montre l'activité reconnue de l'AMPK de type sauvage ; et un dérivé comprend ladite sous-unité γ, un homologue, ou un mutant comme défini ci-dessus, fusionné à un autre polypeptide ;
b) la mise en contact d'un polypeptide d'une sous-unité γ, d'un fragment, d'un homologue, d'un dérivé ou d'un mutant de celle-ci, avec une substance test dans des conditions qui permettraient à du 5'-AMP et/ou de l'ATP de se lier au polypeptide d'une sous-unité γ; et
c) la détermination si ladite substance test s'est liée à un polypeptide d'une sous-unité γ ou un fragment, un homologue, un dérivé ou un mutant de celle-ci.

2. Procédé selon la revendication 1 comprenant en outre l'étape de l'administration d'une substance, dont on a déterminé qu'elle était capable de se lier au polypeptide d'une sous-unité γ de l'AMPK, à l'AMPK, afin de déterminer un quelconque effet modulateur de la substance sur l'activité de l'AMPK.

3. Procédé selon la revendication 2 d'identification d'un agoniste de l'AMPK.

4. Procédé selon la revendication 2 d'identification d'un antagoniste de l'AMPK.

5. Procédé selon l'une quelconque des revendications précédentes dans lequel la sous-unité γ est la sous unité γ1, γ2 et/ou γ3 de l'AMPK.

6. Procédé selon l'une quelconque des revendications précédentes dans lequel un fragment de la sous-unité γ est utilisé.

7. Procédé selon la revendication 6 dans lequel le fragment comprend au moins un domaine CBS.

8. Procédé selon la revendication 7 dans lequel le fragment comprend des domaines CBS 1 et 2,3 et 4 ou 1 à 4.

9. Procédé selon l'une quelconque des revendications précédentes dans lequel la sous-unité γ ou un fragment est une sous-unité γ2 ou un fragment et comprend une ou plusieurs mutations, R302Q, H383R, T400N, L*ins* et/ou R531G.

10. Procédé selon l'une quelconque des revendications précédentes dans lequel la sous-unité γ est AMPK sous la forme d'une protéine de fusion.

11. Procédé selon l'une quelconque des revendications précédentes dans lequel la sous-unité γ, un fragment, un dérivé, un homologue ou un mutant de celle-ci a été produit de manière recombinante.

12. Procédé selon l'une quelconque des revendications précédentes comprenant en outre le fait d'identifier si la substance est capable de se lier à un autre domaine de l'AMPK.

13. Procédé selon la revendication 12 dans lequel le domaine de l'AMPK est le domaine kinase.

14. Procédé selon l'une quelconque de revendications précédentes dans lequel l'agent test est choisi pour se lier de manière sélective au domaine d'une sous-unité γ de préférence à un quelconque autre domaine.

15. Procédé selon l'une quelconque des revendications précédentes dans lequel l'agent test est choisi pour se lier de manière sélective à la sous-unité γ d'AMPK plus qu'à un ou plusieurs domaines CBS d'une autre protéine.

16. Procédé selon la revendication 15 dans lequel l'autre protéine est une IMP déshydrogénase, des protéines de canal chlorure CLC et/ou une cystathionine β-synthase.

17. Procédé d'identification d'une substance capable de se lier à un domaine CBS d'une protéine possédant un tel ou de tels domaines CBS, ou un fragment, un dérivé, un homologue, ou un mutant de celui-ci, lequel procédé comprend ;
a) la fourniture un polypeptide d'une sous-unité à domaine CBS ou un fragment, un homologue, un dérivé ou un mutant de celui-ci, dans lequel ledit fragment est une partie d'une sous-unité CBS, qui est capable de se lier à du 5'-AMP et/ou de l'ATP ; un homologue est un variant d'une sous-unité CBS d'AMPK d'une espèce différente ; un mutant comprend une délétion, une substitution, une insertion ou une inversion d'un ou plusieurs acides aminés dans la séquence de ladite sous-unité CBS de l'AMPK mais dont le mutant de l'AMPK montre l'activité reconnue de l'AMPK de type sauvage ; et un dérivé comprend ladite sous-unité γ, un homologue, ou un mutant comme défini ci-dessus, Fusionné à un autre polypeptide ;
b) la mise en contact d'un polypeptide d'une sous-unité à domaine CBS, ou d'un fragment, d'un homologue, d'un dérivé ou d'un mutant de celui-ci, avec une substance test dans des conditions qui permettraient à du 5'-AMP et/ou de l'ATP de se lier au polypeptide d'une sous-unité à domaine CBS ; et
c) la détermination si ladite substance test s'est liée à un polypeptide d'une sous-unité à domaine CBS.
